# EUROPÄISCHE PATENTSCHRIFT

(11) **EP 4 070 844 B1**
(45) Veröffentlichungstag und Bekanntmachung des Hinweises auf die Patenterteilung: **06.08.2025**
(21) Anmeldenummer: 22166756.1
(22) Anmeldetag: 05.04.2022
(51) Int. Cl.: A61M 16/06

(54) **ATEMMASKE MIT FÜHRUNGSBEREICH**
BREATHING MASK WITH GUIDE AREA
MASQUE RESPIRATOIRE POURVU DE ZONE DE GUIDAGE

(30) Priorität: 09.04.2021 DE 102021001835
(43) Veröffentlichungstag der Anmeldung: 12.10.2022
(73) Patentinhaber: Löwenstein Medical Technology S.A., 2557 Luxembourg (LU)
(72) Erfinder: Bechtel, Martin, 21423 Winsen / Luhe (DE); Frerichs, Arnold, 21614 Buxtehude (DE); Gardein, Joachim, 38430 Icod de los Vinos (ES)
(74) Vertreter: Löwenstein Medical IP

(56) Entgegenhaltungen:
- WO-A2-2017/068530
- DE-A1- 102005 041 716
- DE-U1- 202004 021 829
- DE-U1- 202005 021 927

## Beschreibung

Atemmasken werden zur Beatmung, zur Unterstützung der Atmung oder als Schutzmaske gegen Aerosole aus festen oder flüssigen Partikeln eingesetzt. Für Atemmasken werden unterschiedlichste Materialen verwendet, die gasdurchlässig oder gasundurchlässig sind, wobei Atemmasken aus gasundurchlässigem Material einen Bereich aufweisen, der partiell aus einem gasdurchlässigen Material besteht oder der einen Auslass für ein Schlauchanschlusssystem für die Einatmung und/oder Ausatmung aufweist.

Atemmasken für die Beatmung oder Atemunterstützung bilden die Schnittstelle zwischen einem Anwender oder Patienten und einem Beatmungsgerät und müssen hohen Anforderungen an Stabilität, Sicherheit und Komfort genügen und gleichzeitig einfach in der Handhabung sein. Atemmasken werden üblicherweise über eine Bänderung am Kopf des Anwenders oder Patienten fixiert. Insbesondere bei einer Heimbeatmung ist der Anwender selbst für das korrekte Anlegen und die Fixierung der Atemmaske am Gesicht verantwortlich. Die Koppelung der Bänderung an die Atemmaske erfolgt üblicherweise über mehrere Koppelpunkte und stellt mitunter hohe Anforderungen an die Fingerfertigkeit des Anwenders oder Patienten. Eine korrekt ausgeführte Befestigung der Atemmaske über die Bänderung ist die Voraussetzung für eine sichere und effiziente Beatmung.

DE202005021927U1 offenbart ein Maskensystem mit Stirnstütze und einem Rahmen, an dem ein mehrteiliges Kopfband befestigt werden kann, wobei untere Kopfbänder mithilfe von Clips am Rahmen befestigt werden können. DE202004021829U1 zeigt ein Maskensystem ohne Stirnstütze und mit einem Rahmen, an dem ein Kopfband über Öffnungen und Zapfen befestigt werden kann. WO2017/068530A2 offenbart ein Patienteninterface mit integriertem Gebläse und DE202005041716A1 offenbart eine Atemmaske mit einem modularen System, wobei die Bauteile kodiert miteinander verbunden sind. EP3417900 beschreibt eine Atemmaske mit einem Maskenkörper und zwei Flügel, die je eine Federplatte 18 mit Hilfe ein Rastzapfen 20 fixiert.

Die Aufgabe der Erfindung besteht darin, eine verbesserte Atemmaske zur Verfügung zu stellen, die einfach und sicher anzulegen ist.

Diese Aufgabe wird erfindungsgemäß gelöst durch eine Atemmaske, das die kennzeichnenden Merkmale des Anspruchs 1 aufweist. Die Unteransprüche betreffen vorteilhafte Ausgestaltungen der Erfindung.

Beschrieben ist eine Atemmaske mit einem Gesichtsteil, das mindestens eine Symmetrieebene, eine umlaufende Dichtung zum Anlegen an das Gesicht eines Patienten und einen Auslass umfasst, wobei das Gesichtsteil eine Außenseite aufweist, die bei Verwendung der Atemmaske von dem Gesicht des Patienten abgewandt ist, wobei das Gesichtsteil mindestens zwei Ankerpunkten aufweist, die beidseitig der Symmetrieebene angeordnet sind und wobei die mindestens zwei Ankerpunkte jeweils ein Aufnahmeelement zur lösbaren Verbindung mit einem Verbindungselement umfassen und wobei das Verbindungselement zur Aufnahme einer Bänderung ausgebildet ist. Dabei ist benachbart zu dem Aufnahmeelement mindestens ein Führungsbereich zur Führung des Verbindungselementes zu dem Aufnahmeelement angeordnet, der durch eine Vertiefung in der Außenseite des Gesichtsteils als Führungsrinne ausgebildet ist.

Die Atemmaske ist alternativ auch dadurch gekennzeichnet, dass der Führungsbereich beidseitig der Symmetrieebene auf dem Gesichtsteil angeordnet ist.

Die Atemmaske ist alternativ auch dadurch gekennzeichnet, dass der Führungsbereich durch Erhebungen auf der Außenseite des Gesichtsteils ausgebildet ist.

Die Atemmaske ist alternativ auch dadurch gekennzeichnet, dass die Führungsrinne über 5 % bis 100 % der Länge des Gesichtsteils verläuft, bevorzugt über 50 % bis 100 %, beispielsweise über 95 % der Länge des Gesichtsteils.

Die Atemmaske ist alternativ auch dadurch gekennzeichnet, dass die Führungsrinne einen Rinneneinlauf umfasst, der durch eine sukzessive Vertiefung einen Übergang von der Außenseite zur endgültigen Vertiefung bildet.

Die Atemmaske ist alternativ auch dadurch gekennzeichnet, dass der Rinneneinlauf an einem oberen Ende des Gesichtsteils angeordnet ist.

Die Atemmaske ist alternativ auch dadurch gekennzeichnet, dass die Führungsrinne einen Rinnenboden, einen hinteren Rinnenrand, einen vorderen Rinnenrand und einen Endbereich umfasst, wobei der Endbereich ein Bereich des Rinnenbodens ist, der von mindestens einem der Rinnenränder umschlossen ist.

Die Atemmaske ist alternativ auch dadurch gekennzeichnet, dass die Oberfläche des Rinnenbodens glatt und/oder strukturiert ausgebildet ist.

Die Atemmaske ist alternativ auch dadurch gekennzeichnet, dass die Oberfläche der Rinnenränder glatt und/oder strukturiert ausgebildet ist.

Die Atemmaske ist alternativ auch dadurch gekennzeichnet, dass die Führungsrinne seitlich begrenzt ist durch die Rinnenränder, wobei die Rinnenränder zwischen der Außenseite und dem Rinnenboden angeordnet sind.

Die Atemmaske ist alternativ auch dadurch gekennzeichnet, dass die Rinnenränder von der Außenseite zum Rinnenboden verlaufend gerade oder gewölbt ausgebildet sind.

Die Atemmaske ist alternativ auch dadurch gekennzeichnet, dass der hintere Rinnenrand mit einem Winkel α1 zu dem Rinnenboden angeordnet ist und der vordere Rinnenrand mit einem Winkel α2 zu dem Rinnenboden angeordnet ist, wobei die Größe der Winkel α1, α2 unabhängig voneinander gewählt werden können.

Die Atemmaske ist alternativ auch dadurch gekennzeichnet, dass der Übergang vom Rinnenboden zu den Rinnenrändern eckig und/oder abgerundet ausgebildet ist.

Die Atemmaske ist alternativ auch dadurch gekennzeichnet, dass der hintere Rinnenrand mit einem Winkel β1 zu der Außenseite angeordnet ist und der vordere Rinnenrand mit einem Winkel β2 zu der Außenseite angeordnet ist, wobei die Größe der Winkel β1, β2 unabhängig voneinander gewählt werden können.

Die Atemmaske ist alternativ auch dadurch gekennzeichnet, dass der Übergang von der Außenseite zu den Rinnenrändern eckig und/oder abgerundet ausgebildet ist.

Die Atemmaske ist alternativ auch dadurch gekennzeichnet, dass die Höhe der Rinnenränder konstant und/oder unterschiedlich hoch ausgebildet ist.

Die Atemmaske ist alternativ auch dadurch gekennzeichnet, dass die Vertiefung der Führungsrinne konstant und/oder unterschiedlich tief ausgebildet ist, wobei die Vertiefung in einem Bereich von 0,5 mm bis 6 mm tief ausgebildet ist.

Die Atemmaske ist alternativ auch dadurch gekennzeichnet, dass die Vertiefung unterschiedlich tief ausgebildet ist, wobei die Vertiefung im Endbereich zwischen 1 mm und 6 mm tief ausgebildet ist, bevorzugt 4 mm tief, wobei die Vertiefung in den anderen Bereichen der Führungsrinne im Wesentlichen 0,8 mm bis 2 mm tief ausgebildet ist, bevorzugt 1 mm tief.

Die Atemmaske ist alternativ auch dadurch gekennzeichnet, dass die Führungsrinne eine Rinnenbreite umfasst, die konstant breit ausgebildet ist und/oder unterschiedliche Breiten aufweist.

Die Atemmaske ist alternativ auch dadurch gekennzeichnet, dass die Führungsrinne unterschiedliche Rinnenbreiten aufweist, wobei ein erster Abschnitt als ein Rinnenkanal mit einer konstanten Rinnenbreite A ausgebildet ist und wobei die Rinnenbreite nach dem Rinnenkanal über eine Rinnenbreite B bis zu einer Rinnenbreite C sukzessive zunimmt und wobei die Rinnenbreite nach Erreichen der Rinnenbreite C über eine Rinnenbreite D bis zu einer Rinnenbreite E sukzessive abnimmt.

Die Atemmaske ist alternativ auch dadurch gekennzeichnet, dass der vordere Rinnenrand und der hintere Rinnenrand zwischen der Rinnenbreite B und der Rinnenbreite C auseinanderlaufen.

Die Atemmaske ist alternativ auch dadurch gekennzeichnet, dass der vordere Rinnenrand und der hintere Rinnenrand zwischen der Rinnenbreite C und der Rinnenbreite E aufeinander zulaufen.

Die Atemmaske ist alternativ auch dadurch gekennzeichnet, dass im Bereich der Rinnenbreite C der hintere Rinnenrand maximal entfernt von der Symmetrieebene angeordnet ist und der vordere Rinnenrand maximal dicht an der Symmetrieebene angeordnet ist.

Die Atemmaske ist alternativ auch dadurch gekennzeichnet, dass der hintere Rinnenrand in einem Bereich zwischen den Rinnenbreiten B und D derart bogenartig zurückweicht, dass die Führungsrinne in diesem Bereich als Rinnenbucht ausgebildet ist, die den Endbereich umfasst.

Die Atemmaske ist alternativ auch dadurch gekennzeichnet, dass der Endbereich maximal entfernt von der Symmetrieebene angeordnet ist.

Die Atemmaske ist alternativ auch dadurch gekennzeichnet, dass die Rinnenbucht vom hinteren Rinnenrand umschlossen ist, wobei eine Rinnenbucht-Weite im horizontalen Verlauf mit zunehmender Entfernung zur Symmetrieebene sukzessive abnimmt.

Die Atemmaske ist alternativ auch dadurch gekennzeichnet, dass die Rinnenbucht eine erste Rinnenbucht-Weite, eine zweite Rinnenbucht-Weite und eine dritte Rinnenbucht-Weite umfasst, die durch den horizontalen Verlauf des hinteren Rinnenrandes ausgebildet sind, wobei die erste Rinnenbucht-Weite größer als die zweite Rinnenbucht-Weite ist und wobei die zweite Rinnenbucht-Weite größer als die dritte Rinnenbucht-Weite ist.

Die Atemmaske ist alternativ auch dadurch gekennzeichnet, dass die erste Rinnenbucht-Weite durch den horizontalen Verlauf des hinteren Rinnenrandes zwischen der Rinnenbreite B und der Rinnenbreite D ausgebildet ist.

Die Atemmaske ist alternativ auch dadurch gekennzeichnet, dass zwischen zwei vorderen Steg-Ansätzen die zweite Rinnenbucht-Weite ausgebildet ist.

Die Atemmaske ist alternativ auch dadurch gekennzeichnet, dass zwischen einem oberen Mittelsteg-Ansatz und einem unteren Mittelsteg-Ansatz die dritte Rinnenbucht-Weite ausgebildet ist.

Die Atemmaske ist alternativ auch dadurch gekennzeichnet, dass der Endbereich in der Rinnenbucht zwischen der zweiten Rinnenbucht-Weite und der dritten Rinnenbucht-Weite angeordnet ist.

Die Atemmaske ist alternativ auch dadurch gekennzeichnet, dass die Höhe des hinteren Rinnenrandes im Bereich des Endbereichs zunehmen kann.

Die Atemmaske ist alternativ auch dadurch gekennzeichnet, dass der Endbereich der Führungsrinne räumlich benachbart zu dem Aufnahmeelement angeordnet ist, wobei das Aufnahmeelement den Endbereich der Führungsrinne überspannt.

Die Atemmaske ist alternativ auch dadurch gekennzeichnet, dass das Aufnahmeelement den Endbereich der Führungsrinne derart überspannt, dass ein Aufnahmeraum entsteht.

Die Atemmaske ist alternativ auch dadurch gekennzeichnet, dass das Aufnahmeelement ein integraler Bestandteil der Außenseite und der Rinnenränder ist.

Die Atemmaske ist alternativ auch dadurch gekennzeichnet, dass das Aufnahmeelement ein integraler Bestandteil der Außenseite und des hinteren Rinnenrandes ist.

Die Atemmaske ist alternativ auch dadurch gekennzeichnet, dass das Gesichtsteils aus einem harten Kunststoff gefertigt ist ausgewählt aus der Gruppe der Polyamide, Polycarbonate, Polyoxymethylene, Polysulfone und Polypropylene.

Die Atemmaske ist alternativ auch dadurch gekennzeichnet, dass das Gesichtsteils aus Polyamid PA12 gefertigt ist.

Die Atemmaske ist alternativ auch dadurch gekennzeichnet, dass das Aufnahmeelement aus demselben Werkstoff wie das Gesichtsteil gefertigt ist.

Die Atemmaske ist alternativ auch dadurch gekennzeichnet, dass Aufnahmeelement, Außenseite und hinterer Rinnenrand einstückig ausgebildet sind.

Die Atemmaske ist alternativ auch dadurch gekennzeichnet, dass das Aufnahmeelement zwei Stege, eine Brücke, einen Aufnahme-Durchmesser und eine Aufnahme-Öffnung umfasst.

Die Atemmaske ist alternativ auch dadurch gekennzeichnet, dass die Stege über Steg-Ansatzflächen, die den vorderen Steg-Ansatz und einen hinteren Steg-Ansatz umfassen, mit dem hinteren Rinnenrand verbunden sind, wobei die Stege räumlich getrennt vom Rinnenboden angeordnet sind.

Die Atemmaske ist alternativ auch dadurch gekennzeichnet, dass die Stege gerade und/oder gebogen ausgebildet sind.

Die Atemmaske ist alternativ auch dadurch gekennzeichnet, dass die Stege mit einem Winkel ε zum Rinnenboden angeordnet sind, wobei der Wert des Winkels ε in einem Bereich von 30° bis 90° liegt, bevorzugt in einem Bereich von 30° bis 60°.

Die Atemmaske ist alternativ auch dadurch gekennzeichnet, dass die Brücke einstückig mit den Stegen gefertigt ist und wobei die Brücke räumlich getrennt vom Rinnenboden angeordnet ist.

Die Atemmaske ist alternativ auch dadurch gekennzeichnet, dass die Stege, die Brücke und der Rinnenboden den Aufnahmeraum begrenzen.

Die Atemmaske ist alternativ auch dadurch gekennzeichnet, dass die Brücke zumindest abschnittsweise kreisrund ausgebildet ist mit einem Aufnahme-Durchmesser und wobei die Brücke unterbrochen ist durch eine Aufnahme-Öffnung.

Die Atemmaske ist alternativ auch dadurch gekennzeichnet, dass die Brücke halbkreisförmig ausgebildet ist.

Die Atemmaske ist alternativ auch dadurch gekennzeichnet, dass das Aufnahmeelement mindestens einen Mittelsteg und mindestens einen, bevorzugt zwei Durchbrüche umfasst, wobei der Mittelsteg zwischen der Brücke und der Außenseite angeordnet ist
Die Atemmaske ist alternativ auch dadurch gekennzeichnet, dass der Mittelsteg auf der Hälfte der Brücke angeordnet ist.

Die Atemmaske ist alternativ auch dadurch gekennzeichnet, dass der mindestens eine Mittelsteg über eine Mittelsteg-Ansatzfläche, die den oberen Mittelsteg-Ansatz und den unteren Mittelsteg-Ansatz umfasst, mit dem hinteren Rinnenrand verbunden ist, wobei der Mittelsteg räumlich getrennt vom Rinnenboden angeordnet ist.

Die Atemmaske ist alternativ auch dadurch gekennzeichnet, dass die Aufnahme-Öffnung kleiner ausgebildet ist als der Aufnahme-Durchmesser.

Die Atemmaske ist alternativ auch dadurch gekennzeichnet, dass die Aufnahme-Öffnung nach vorne ausgerichtet ist.

Die Atemmaske ist alternativ auch dadurch gekennzeichnet, dass das Verbindungselement ein lösbares Teil der Atemmaske ist.

Die Atemmaske ist alternativ auch dadurch gekennzeichnet, dass das Verbindungselement aus demselben Werkstoff wie das Gesichtsteil gefertigt ist.

Die Atemmaske ist alternativ auch dadurch gekennzeichnet, dass das Verbindungselement ein Befestigungselement und einen Haltesteg umfasst, wobei das Befestigungselement und der Haltesteg an sich gegenüberliegenden Seiten des Verbindungselements angeordnet sind.

Die Atemmaske ist alternativ auch dadurch gekennzeichnet, dass eine Grundfläche des Verbindungselements eine Aussparung umfasst, die von der inneren Grundfläche bis zur äußeren Grundfläche verläuft, wodurch der Haltesteg gebildet wird.

Die Atemmaske ist alternativ auch dadurch gekennzeichnet, dass die Aussparung halbmondförmig ausgebildet ist.

Die Atemmaske ist alternativ auch dadurch gekennzeichnet, dass der Haltesteg ausgebildet ist, eine Bänderung aufzunehmen, wobei die Bänderung ausgebildet ist, die Atemmaske am Kopf eines Anwenders und/oder Patienten zu befestigen.

Die Atemmaske ist alternativ auch dadurch gekennzeichnet, dass das Befestigungselement eine Knopfplatte, einen Überhang und einen Hals umfasst, wobei die Knopfplatte über den Hals an dem Verbindungselement angeformt ist.

Die Atemmaske ist alternativ auch dadurch gekennzeichnet, dass die Knopfplatte und der Hals rund oder gerundet und mit jeweils einem maximalen Durchmesser ausgeführt sind, wobei der maximale Durchmesser der Knopfplatte größer ist als der maximale Durchmesser des Halses.

Die Atemmaske ist alternativ auch dadurch gekennzeichnet, dass das Aufnahmeelement eingerichtet und ausgebildet ist, das Verbindungselement über das an dem Verbindungselement angeformte Befestigungselement lösbar aufzunehmen.

Die Atemmaske ist alternativ auch dadurch gekennzeichnet, dass die Aufnahme-Öffnung eingerichtet und ausgebildet ist, den Hals aufzunehmen und in den Aufnahme-Durchmesser zu führen.

Die Atemmaske ist alternativ auch dadurch gekennzeichnet, dass der Aufnahmeraum eingerichtet und ausgebildet ist, die Knopfplatte aufzunehmen.

Die Atemmaske ist alternativ auch dadurch gekennzeichnet, dass das Verbindungselement durch Aufnahme des Befestigungselements in den Aufnahme-Durchmesser und den Aufnahmeraum reversibel mit dem Aufnahmeelement verbunden ist.

Die Atemmaske ist alternativ auch dadurch gekennzeichnet, dass die Verbindung des Verbindungselements an das Aufnahmeelement beweglich gelagert ist, insbesondere drehbeweglich.

Die Atemmaske ist alternativ auch dadurch gekennzeichnet, dass das Verbindungselement eine Seitenwand umfasst, wobei die Seitenwand mindestens eine, bevorzugt zwei Griffmulden umfasst, die breiter ausgebildet sind als die Seitenwand.

Die Atemmaske ist alternativ auch dadurch gekennzeichnet, dass die mindestens eine Griffmulde eine Griffmulden-Innenwand und eine Griffmulden-Außenwand umfasst, wobei die Griffmulden-Außenwand mindestens ein Strukturelement umfasst, das in Form von Rippen, Wellen, Gitterstrukturen, Netzen und/oder Punkten, beispielsweise in Form von rippenartigen Erhöhungen, ausgebildet ist.

### Figurenliste

In den Figuren sind Ausführungsbeispiele der erfindungsgemäßen Atemmaske 100 dargestellt. Es zeigen:
Fig. 1 eine Atemmaske 100 seitlich von vorne 520.
Fig. 2 eine Atemmaske 100 ohne Verbindungselement 80 in einer Draufsicht von vorne 520.
Fig. 3 eine Atemmaske 100 ohne Verbindungselement 80 von links 210.
Fig. 4 ein Gesichtsteil 30 von vorne 520 zur Verdeutlichung der hierin verwendeten Richtungen und Lagebeziehungen.
Fig. 5 ein Gesichtsteil 30 von oben 310.
Fig. 6 ein Gesichtsteil 30 von unten 320.
Fig. 7 ein Gesichtsteil 30 in einer Draufsicht von vorne 520.
Fig. 8 einen Ausschnitt des Gesichtsteils 30 in einer Draufsicht von vorne 520.
Fig. 9 eine perspektivische Ansicht eines Ausschnittes eines Gesichtsteils 30 von schräg oben 310.
Fig. 10 und 11 schematische Querschnitte einer Führungsrinne 50.
Fig. 12 eine schematische Übersicht eines hinteren Rinnenrandes 51 im Bereich einer Rinnenbucht 54 in einer Draufsicht.
Fig. 13 einen Ausschnitt eines Gesichtsteils 30 in einer Draufsicht zur Darstellung eines Aufnahmeelements 70.
Fig. 14 ein Aufnahmeelement 70 im Detail in einer Draufsicht.
Fig. 15 eine schematische Abbildung eines Aufnahmeelements 70 von vorne 520.
Fig. 16 einen Ausschnitt eines Gesichtsteils 30 von vorne 520 zur Darstellung eines Aufnahmeelements 70 von vorne 520.
Fig. 17 ein Verbindungselement 80 in einer Draufsicht von hinten 420.
Fig. 18 ein Verbindungselement 80 in einer perspektivischen Ansicht schräg von vorne 520.
Fig. 19 ein Verbindungselement 80 von der Seite.
Fig. 20 das Befestigungselement 90 im Detail.
Fig. 21 ein Verbindungselement 80, das über das Befestigungselement 90 mit dem Aufnahmeelement 70 verbunden ist.

### Ausführungsbeispiele

Die erfindungsgemäße Atemmaske 100 kann eine Maske zur Beatmung, eine Maske zur Atemunterstützung oder eine Schutzmaske sein. Die Atemmaske 100 kann eine Mund-Nasen-Schutzmaske, eine Nasalmaske oder eine Vollgesichtsmaske sein. In den nachfolgenden Ausführungsbeispielen ist eine Vollgesichtsmaske zur Beatmung gezeigt.

Weitere Merkmale und Vorteile der vorliegenden erfindungsgemäßen Atemmaske 100 werden in den nachfolgenden Beschreibungen von Ausführungsbeispielen anhand der Figuren deutlich. Die Erfindung ist nicht auf die dargestellten Ausführungsbeispiele beschränkt.

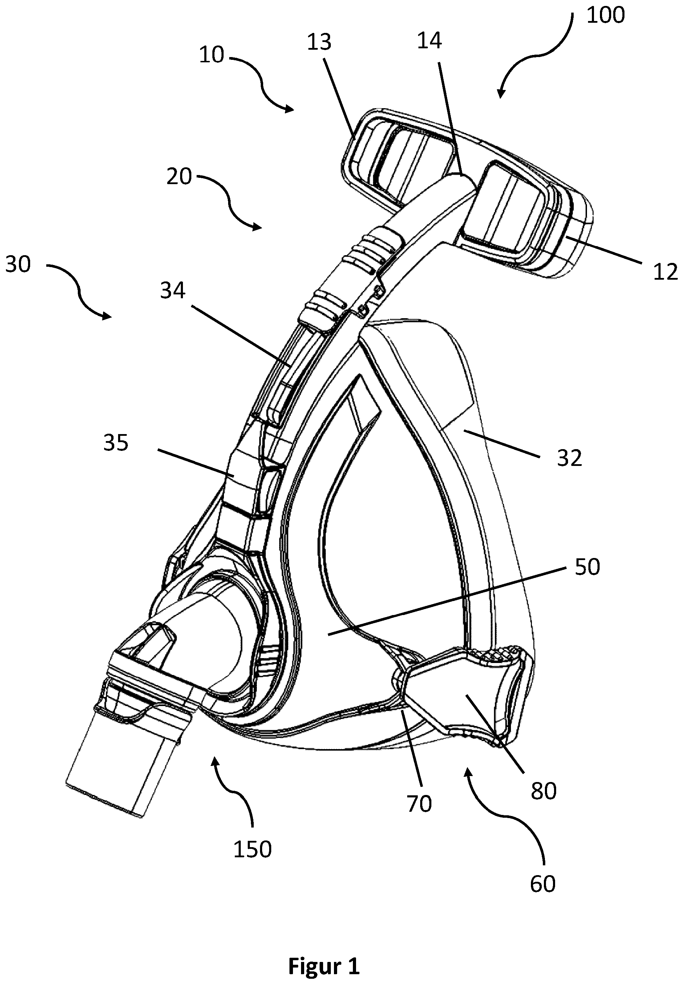

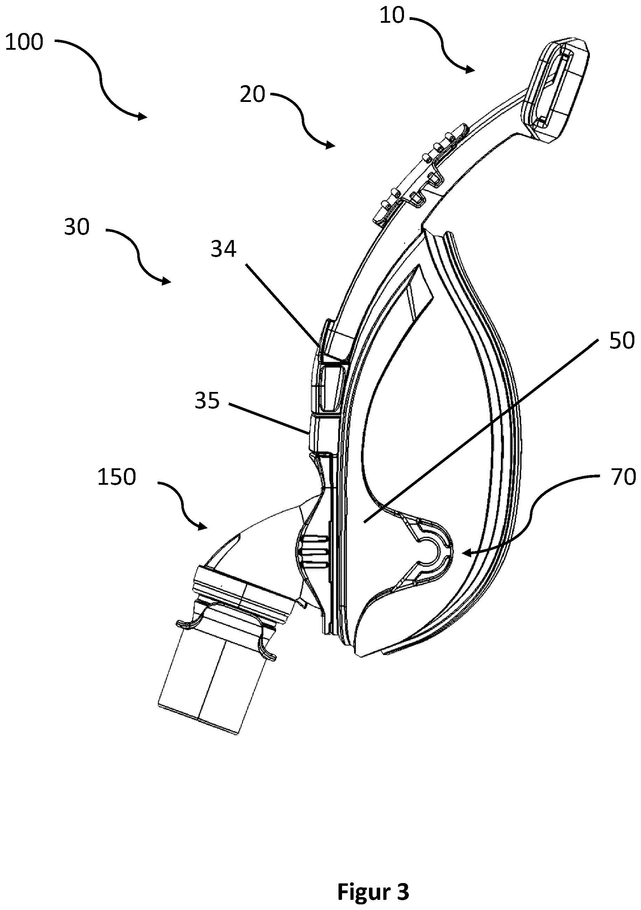

**Fig. 1 bis 3** zeigen aus unterschiedlichen Perspektiven eine Atemmaske 100 zur Atmung und/oder Beatmung mit einem Stirnteil 10, einem Übergangsteil 20 und einem Gesichtsteil 30 sowie einem Schlauchanschlusssystem 150.

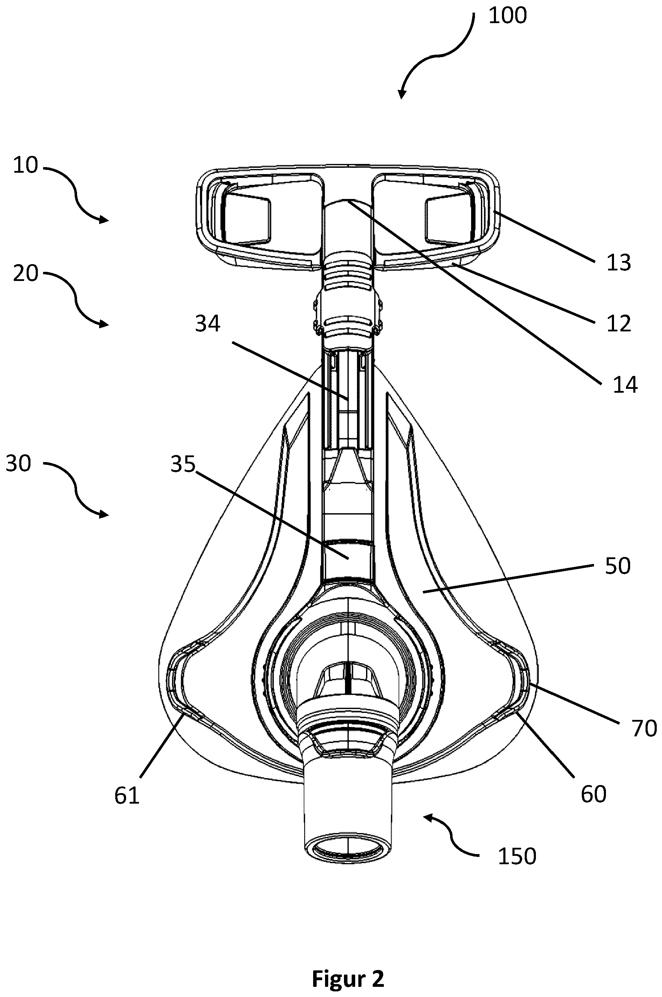

Das Stirnteil 10 umfasst ein Stirnpolster 12, mindestens einen oberen Ankerpunkt 13 und eine Verbindungsstelle 14 (siehe Fig. 1 und 2).

Das Übergangsteil 20 kann das Stirnteil 10 über die Verbindungsstelle 14 mit dem Gesichtsteil 30 verbinden. Das Übergangsteil 20 kann längsverstellbar und beweglich gelagert sein.

Das Gesichtsteil 30 kann eine umlaufende Dichtung 32 (nur in Fig. 1 dargestellt), eine Koppelstelle 34, ein Zwischenglied 35, einen Auslass 36 (nicht gezeigt), einen Führungsbereich 50 und mindestens zwei Ankerpunkte 60/61 umfassen. Die mindestens zwei Ankerpunkte 60/61 umfassen jeweils ein Aufnahmeelement 70 und ein Verbindungselement 80 (nur in Fig. 1 dargestellt).

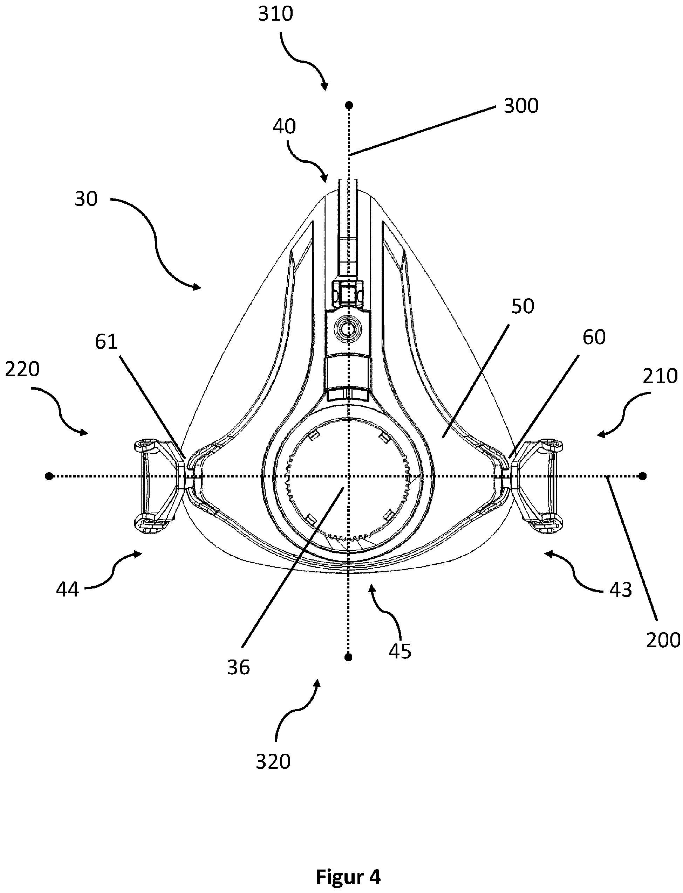

**Fig. 4** zeigt ein Gesichtsteil 30 in einer Draufsicht von vorne 520. Das Gesichtsteil 30 weist in üblicher Bauweise eine annähernde Dreiecksform auf. Die Dreiecksform des Gesichtsteils 30 weist vorzugsweise gerundete Ecken bzw. Spitzen auf. Die Dreiecksform weist vorzugsweise keine Geraden auf, sondern ist leicht nach außen gewölbt ausgebildet.

Eine Spitze 40 des Dreiecks wird als oberes Ende oder oben 310 liegend definiert. Die Spitze 40 des Dreiecks ist in einer 12-Uhr-Position angeordnet und ist ausgebildet zur Anlage auf dem Nasenrücken. Die beiden anderen Eckpunkte des Dreiecks werden als untere Eckpunkte 43/44 definiert.

Eine Basis 45 des Dreiecks verbindet die beiden unterem Eckpunkte 43/44 und wird als unteres Ende oder unten 320 liegend definiert. Die Basis 45 des Dreiecks ist ausgebildet zur Anlage auf den Bereich zwischen Unterlippe und Kinn.

Die unteren Eckpunkte 43/44 sind in einer 3 bzw. 9-Uhr-Position angeordnet, wenn ein unten beschriebener Auslass 36 als Mittelpunkt eines gedachten Ziffernblattes angesehen wird. Der Eckpunkt, der bei Betrachtung der Außenseite 31 in einer 3-Uhr Position angeordnet ist wird als linker unterer Eckpunkt 43 definiert. Der Eckpunkt, der bei Betrachtung der Außenseite 31 in einer 9-Uhr Position angeordnet ist wird als rechter unterer Eckpunkt 44 definiert.

Zu besseren Anschaulichkeit werden hierin eine horizontale Ebene 200 und eine vertikal verlaufende Symmetrieebene 300 festgelegt. Die horizontale Ebene 200 verläuft waagerecht. Die horizontale Ebene 200 definiert die maximale Breite des Gesichtsteils 30.

Die maximale Breite des Gesichtsteils 30 liegt in einem Bereich von 80 mm bis 120 mm, bevorzugt von 95 mm bis 100 mm. Beispielsweise beträgt die maximale Breite des Gesichtsteils (30) 98 mm. Im Bereich der maximalen Breite des Gesichtsteils sind die beiden Ankerpunkte 60/61 angeordnet.

Die Symmetrieebene 300 steht senkrecht auf der horizontalen Ebene 200. Das Gesichtsteil 30 ist in diesem Ausführungsbeispiel spiegelsymmetrisch, das bedeutet, dass das Gesichtsteil 30 über die hierin dargestellte Symmetrieebene 300 gespiegelt werden kann.

Die Begriffe der Symmetrieebene 300 und der horizontalen Ebene 200 behalten hierin ihre Gültigkeit, auch wenn die Atemmaske 100 gekippt oder gedreht dargestellt ist und die Ebenen anders erscheinen. Die Symmetrieebene 300 definiert die maximale Länge des Gesichtsteils 30.

Die maximale Länge des Gesichtsteils 30 liegt in einem Bereich von 40 mm bis 200 mm, bevorzugt in einem Bereich von 80 mm bis 140 mm, besonders bevorzugt in einem Bereich von 90 mm bis 130 mm. Beispielsweise kann das Gesichtsteil in drei Größenkategorien ausgebildet sein, wobei die maximale Länge des Gesichtsteils 30 in einer kleinen Ausführungsform beispielsweise in einem Bereich von 90 mm bis 105 mm liegen, in einer mittleren Ausführungsform beispielsweise in einem Bereich von 105 mm bis 116 mm und in einer großen Ausführungsform beispielsweise in einem Bereich von 116 mm bis 130 mm.

Das Verhältnis der maximalen Breite zur maximalen Länge des Gesichtsteils 30 liegt, bei einer vorbestimmten maximalen Maskenbreite von 98 mm, in einer kleinen Ausführungsform beispielsweise in einem Bereich von 1:0,9 bis 1:1,07, in einer mittleren Ausführungsform beispielsweise in einem Bereich von 1:1,07 bis 1:1,18 und in einer großen Ausführungsform beispielsweise in einem Bereich von 1:1,18 bis 1:1,33.

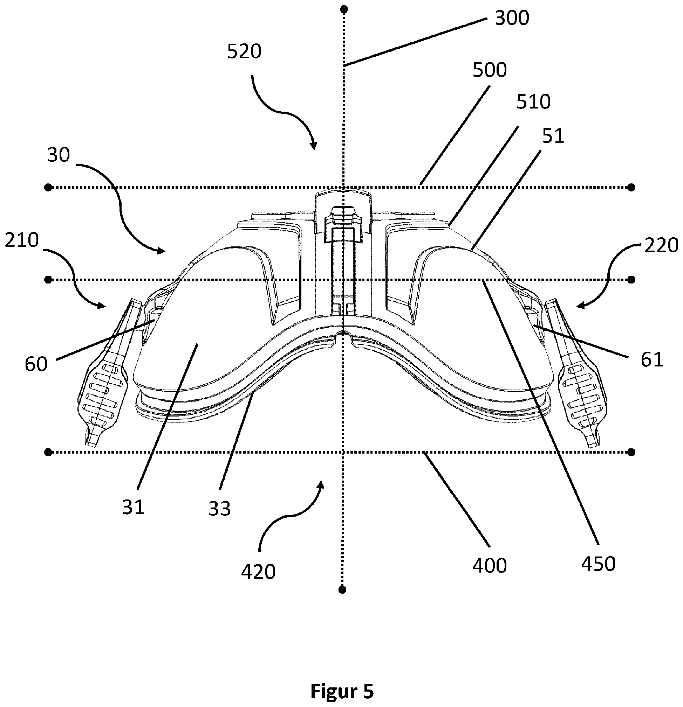

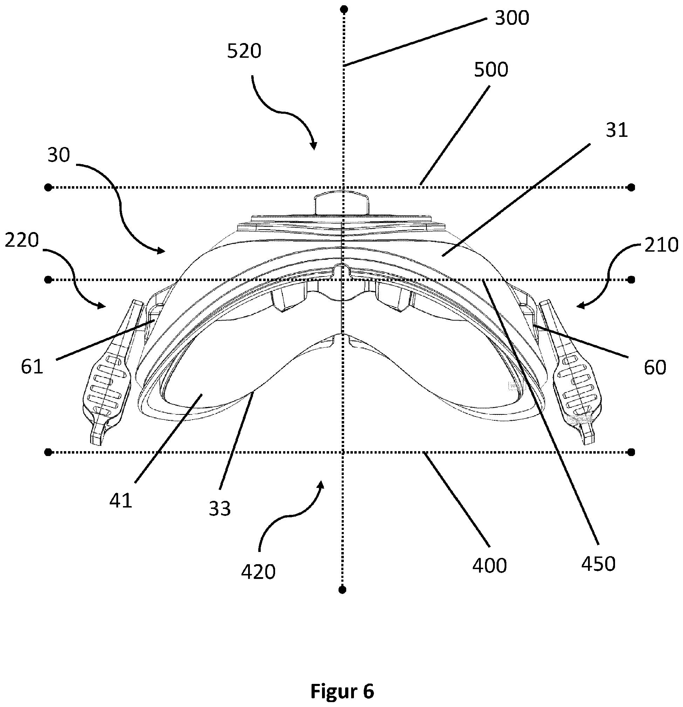

**Fig. 5** zeigt ein Gesichtsteil 30 von oben 310 betrachtet und **Fig. 6** zeigt ein Gesichtsteil 30 von unten 320 betrachtet. Eine vordere Ebene 500, eine mittlere Ebene 450 und eine hintere Ebene 400 sind Schnitte der horizontalen Ebene 200 (Fig. 4). Die Symmetrieebene 300 verläuft senkrecht zu den Ebenen 500/450/300.

Die vordere Ebene 500 ist in einem Benutzungszustand von einem Gesicht des Patienten (nicht gezeigt) abgewandt. Die hintere Ebene 400 entspricht in einem Benutzungszustand der Ebene eines Gesichtes des Patienten (nicht gezeigt). Der Abstand zwischen den Ebenen 400 und 500 definiert die maximale Tiefe des Gesichtsteils 30.

Eine Außenseite 31 des Gesichtsteils 30 ist definitionsgemäß diejenige Seite, die in einem Benutzungszustand von einem Gesicht des Patienten (nicht gezeigt) abgewandt ist. Die Außenseite 31 ist der vorderen Ebene 500 zugewandt und liegt entsprechend vorne 520.

Eine Innenseite 41 des Gesichtsteils 30 ist definitionsgemäß diejenige Seite, die in einem Benutzungszustand einem Gesicht des Patienten (nicht gezeigt) zugewandt ist. Die Innenseite 41 (nur in Fig. 6 gezeigt) ist der hinteren Ebene 400 zugewandt und liegt entsprechend hinten 420.

Das Gesichtsteil 30 ist beispielsweise aus einem Kunststoff gefertigt. Das Gesichtsteil 30 kann beispielsweise über ein Spritzgußverfahren hergestellt werden. Denkbar sind aber auch andere geeignete Werkstoffe und Herstellungsverfahren, die in Bezug auf Stabilität, Festigkeit, Flexibilität, Temperaturbeständigkeit, Gewicht, Kosten, Biokompatibilität, Optik und Komfort geeignet erscheinen.

Das Gesichtsteil 30 ist bevorzugt aus einem harten Kunststoff gefertigt. Geeignete Kunststoffe umfassen beispielsweise Polyamide, Polycarbonate, Polyoxymethylene, Polysulfone und Polypropylene. Beispielsweise ist das Gesichtsteil 30 aus Polyamid PA12 gefertigt. Die Vorteile von Polyamid PA12 bestehen in einer sehr guten Temperaturbeständigkeit, einer hohen Belastbarkeit und einer hohen Transparenz des Werkstoffes.

Der Werkstoff des Gesichtsteils 30 ist im vorliegenden Ausführungsbeispiel ein gasundurchlässiges Material. Es ist aber auch denkbar, dass das Gesichtsteil 30 aus einem gasdurchlässigen Material besteht.

Aus Fig. 5 und 6 wird ersichtlich, dass die Außenseite 31 des Gesichtsteils 30 weitestgehend konvex gewölbt ist und die Innenseite 41 (siehe Fig. 6) des Gesichtsteils 30 weitestgehend konkav gewölbt ist. Durch die Wölbung des Gesichtsteils 30 weist der äußere Rand 33 nach hinten 420 und es entsteht ein Hohlraum zwischen der Oberfläche eines nicht abgebildeten Gesichts und dem Gesichtsteil 30 der Atemmaske 100. In diesem Hohlraum befindet sich in einem Benutzungszustand Atemgas. Der Hohlraum ist bevorzugt so groß ausgebildet, dass die Innenseite 41 des Gesichtsteils 30 nicht die Oberfläche des Gesichts berührt. Zugleich ist der Hohlraum bevorzugt so klein wie möglich ausgebildet, damit der Bereich, in dem sich Atemgas befindet, möglichst gering ist. Ein möglichst geringes Volumen ist vorteilhaft, da so das Volumen, in dem sich Mischgas bzw. CO₂ sammeln kann, möglichst geringgehalten wird.

Aus Fig. 4 wird ersichtlich, dass die Ankerpunkte 60/61 im Bereich der maximalen Breite des Gesichtsteils angeordnet sind. Aus Fig. 5 und 6 wird darüber hinaus ersichtlich, dass die Ankerpunkte 60/61 in diesem Ausführungsbeispiel in einem Bereich zwischen der mittleren Ebene 450 und der hinteren Ebene 400 angeordnet sind.

Vorteilhafterweise liegen die Ankerpunkte 60/61 möglichst weit unten 320 und möglichst weit hinten 420, wodurch die Stabilität der Atemmaske 100 nach Anlage am Gesicht erhöht wird. Die Ankerpunkte 60/61 liegen somit möglichst nahe bei einem nicht gezeigten Gesicht eines Patienten. Die Ankerpunkte 60/61 liegen jedoch nach Anlage am Gesicht so weit entfernt von einem nicht gezeigten Gesicht, dass ein hierin unten beschriebenes Verbindungselement 80 das Gesicht nicht berühren kann.

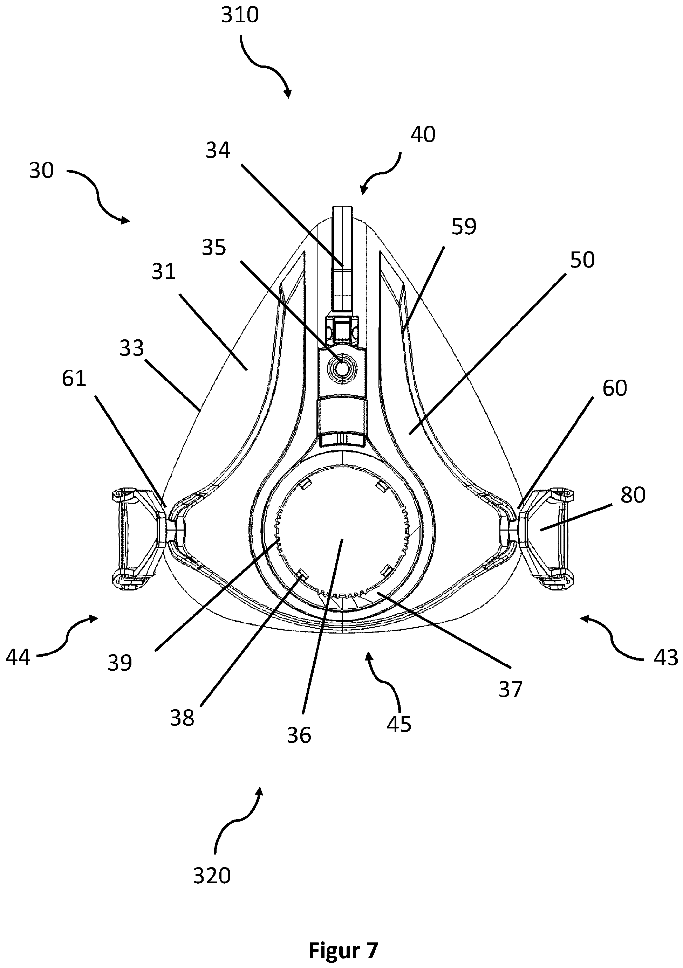

**Fig. 7** zeigt das Gesichtsteil 30 in einer Draufsicht von vorne 520. Das Gesichtsteil 30 umfasst wie oben beschrieben eine Außenseite 31 und eine Innenseite 41 (hier nicht gezeigt). Das Gesichtsteil 30 ist begrenzt durch einen äußeren Rand 33. Der äußere Rand 33 ist ausgebildet, eine nachfolgend beschriebene Dichtung 32 aufzunehmen.

Das Gesichtsteil 30 weist üblicherweise eine Dichtung 32 (siehe Fig. 1) auf, die am äußeren Rand 33 des Gesichtsteils 30 angeordnet ist. Die Dichtung 32 ist durchlaufend am gesamten äußeren Rand 33 des Gesichtsteils 30 angeordnet. Die umlaufende Dichtung 32 ist ausgebildet, um an der Gesichts-Haut eines Anwenders / Patienten anzuliegen. Die umlaufende Dichtung 32 ist flexibel ausgebildet, um sich an das Gesicht des Anwenders / Patienten anzupassen. Die umlaufende Dichtung 32 ist bevorzugt aus einem flexiblen Kunststoff gefertigt, beispielsweise aus einem Silikon. Denkbar sind aber auch andere geeignete Werkstoffe, die in Bezug auf Gasabdichtung, Stabilität, Flexibilität und Komfort geeignet erscheinen.

In diesem Ausführungsbeispiel ist das Gesichtsteil 30 gasabdichtend gegenüber der Umgebungsluft ausgebildet. Ein Gasaustausch kann in diesem Ausführungsbeispiel nur über den unten dargestellten Auslass 36 stattfinden.

An der Spitze 40 des Gesichtsteils 30 kann eine Koppelstelle 34 angeordnet sein. Über die Koppelstelle 34 kann das Gesichtsteil 30 über ein Übergangsteil 20 mit einem Stirnteil 10 verbunden werden. Die Koppelstelle 34 kann das Gesichtsteil 30 mit dem Übergangsteil 20 verbinden. Die Koppelstelle 34 kann über die äußere Oberfläche des Gesichtsteils 30 herausragen und einen Vorsprung bilden. An diesen herausragenden Vorsprung kann ein Übergangsteil 20 angelagert sein. Die Koppelstelle 34 kann beispielsweise als Führungsschiene ausgebildet sein.

Die Koppelstelle 34 kann ein- oder zweiteilig ausgebildet sein. Die Koppelstelle 34 ist bevorzugt einteilig mit dem Gesichtsteil 30 ausgebildet und aus dem gleichen Material wie das Gesichtsteil 30. In anderen Worten ist die Koppelstelle 34 ein fester Bestandteil des Gesichtsteils 30. Es ist aber auch denkbar, dass Gesichtsteil 30 und Koppelstelle 34 zweiteilig ausgebildet sind. Bei einer zweiteiligen Ausführung können Gesichtsteil 30 und Koppelstelle 34 aus demselben Material gefertigt sein. Es ist aber auch denkbar, dass die Koppelstelle 34 aus einem anderen Werkstoff besteht als das Gesichtsteil 30.

An die Koppelstelle 34 nach unten 320 anschließend kann ein Zwischenglied 35 angeordnet sein. In anderen Worten kann ein Zwischenglied 35 zwischen der Koppelstelle 34 und einem nachfolgend beschriebenen Auslass 36 angeordnet sein. In dem Zwischenglied 35 können weitere Funktionselemente angeordnet sein, insbesondere Messstutzen wie beispielsweise ein Druckmessstutzen oder ein CO₂-Messstutzen oder ähnliches (nicht abgebildet).

Unter dem Zwischenglied 35 und zwischen den unteren Eckpunkten 43/44 des Gesichtsteils 30 kann ein Auslass 36 mit einer beispielsweise runden Form angeordnet sein. Der Auslass 36 kann auch jegliche andere Form von rund über oval bis eckig aufweisen. Im Falle einer Schutzmaske kann der Auslass auch großflächig aus einem gasdurchlässigen Material ausgebildet sein. Der Auslass 36 kann als Öffnung oder geschlossen ausgebildet sein.

Der in Fig. 7 dargestellte Auslass 36 ist als kreisförmige Öffnung im Gesichtsteil 30 ausgebildet. Der Auslass 36 umfasst einem Auslass-Rand 37, der ein oder mehrere Kerbeelemente 39 und Einrastelemente 38 aufweisen kann. Über diese Elemente können weitere Anschlusseinheiten in die Öffnung eingebracht werden wie beispielsweise ein Anschlussstutzen zur Aufnahme eines Verbindungsschlauches, mit dem das Gesichtsteil 30 mit einer Atemgasquelle verbunden werden kann. An dem Auslass 36 kann somit ein Schlauchanschlusssystem 150 (siehe Fig. 1-3) angeschlossen werden.

In anderen Ausführungsform ist es auch denkbar, dass der Auslass 36 geschlossen ist. Beispielsweise kann der Auslass 36 aus einem anderen Material als das Gesichtsteil 30 gefertigt sein. Denkbar ist, dass der Auslass 36 aus einem Material gefertigt ist, das einen Gasaustausch zulässt, wohingegen das Gesichtsteil 30 aus einem Material gefertigt ist, das keinen Gasaustausch zulässt.

Das Gesichtsteil 30 umfasst zudem wie nachfolgend beschriebenen mindestens einen Führungsbereich 50 und die mindestens zwei Ankerpunkte 60/61 mit jeweils einem Aufnahmeelement 70 und einem Verbindungselement 80. Der Führungsbereich 50 ist zur Führung eines Verbindungselementes 80 zu einem Aufnahmeelement 70 ausgebildet, und ist beispielsweise als Führungsrinne 50 ausgebildet.

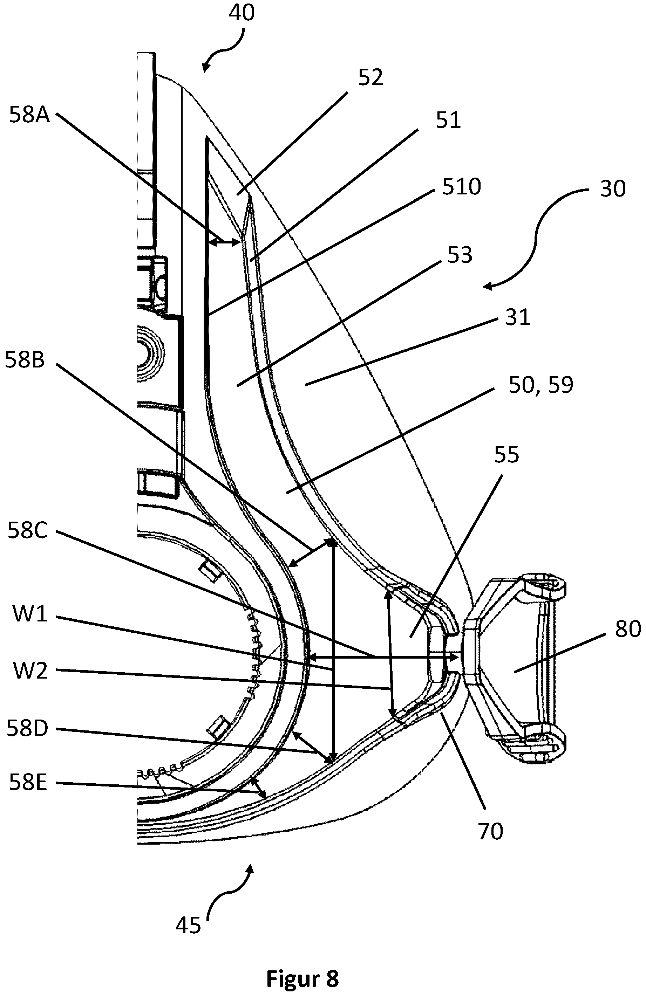

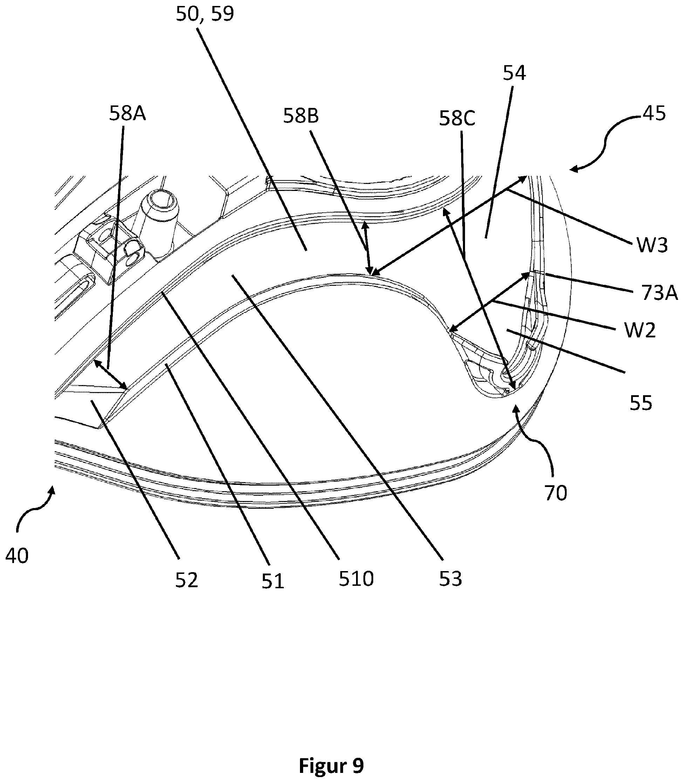

**Fig. 8** zeigt einen Ausschnitt des Gesichtsteils 30 in einer Draufsicht von vorne 520 mit der Außenseite 31 des Gesichtsteils 30, die mindestens eine Führungsrinne 50 umfasst. **Fig. 9** zeigt eine perspektivische Ansicht eines Ausschnittes eines Gesichtsteils 30 von schräg oben 310.

Die Führungsrinne 50 ermöglicht es dem Anwender und /oder dem Patienten, ein unten beschriebenes Verbindungselement 80 entlang der Führungsrinne 50 zu führen. Es ist insbesondere daran gedacht, ein Verbindungselement 80 zu einem nachstehend erläuterten Aufnahmeelement 70 zu führen, das räumlich benachbart zu einem Endbereich 55 (siehe unten) der Führungsrinne 50 angeordnet ist.

Die Führungsrinne 50 kann beispielsweise durch eine Vertiefung 59 in der Außenseite 31 des Gesichtsteils 30 ausgebildet sein. Es ist auch denkbar, dass die Führungsrinne 50 durch Erhebungen auf der Außenseite 31 des Gesichtsteils 30 ausgebildet ist wie beispielsweise durch erhabene Führungskanten oder ähnliches.

Die Führungsrinne 50 ist auf wenigstens einer Seite des Gesichtsteils 30 angeordnet, bevorzugt beidseitig. Besonders bevorzugt ist die Führungsrinne 50 symmetrisch zu beiden Seiten der Symmetrieebene 300 (Fig. 4) ausgebildet.

Fig. 8 und 9 zeigen beispielhaft ein Ausführungsbeispiel des Gesichtsteils 30 mit einer Führungsrinne 50, die als Vertiefung 59 in der Außenseite 31 ausgebildet ist. Die Führungsrinne 50 ist in diesem Ausführungsbeispiel achsensymmetrisch über die Symmetrieebene 300. Im Folgenden wird der Einfachheit halber im Wesentlichen nur eine der symmetrischen Hälften der Führungsrinne 50 beschrieben.

Die Vertiefung 59 der Führungsrinne 50 liegt in einem Bereich von 0,5 mm bis 6 mm, bevorzugt von 0,8 mm bis 2 mm, beispielsweise ist die Führungsrinne 50 im Wesentlichen 1 mm tief ausgebildet. Die Vertiefung 59 kann an speziell ausgebildeten Bereichen der Führungsrinne 50 partiell eine von dem Rest der Führungsrinne 50 abweichende Tiefe aufweisen. Der Grad der Vertiefung 59 ist derart gewählt, dass die Führungsrinne 50 eine sichere und einfache Handhabung für den Anwender und/oder den Patienten bietet.

Die Führungsrinne 50 kann gerade und/oder geschwungen verlaufen. Die Führungsrinne 50 kann über das gesamte Gesichtsteil 30 erstreckt sein oder nur über Teile des Gesichtsteils 30 verlaufen. Die Führungsrinne 50 erstreckt sich über 5 % bis 100 % der Länge des Gesichtsteils 30, bevorzugt über 50 % bis 100 %, beispielsweise über 95 % der Länge des Gesichtsteils 30.

Im dargestellten Ausführungsbeispiel ist die mindestens eine Führungsrinne 50 über nahezu die gesamte Außenseite 31 erstreckt und weitestgehend von der Spitze 40 zur Basis 45 vertikal verlaufend angeordnet.

Die Führungsrinne 50 beginnt an einem Rinneneinlauf 52. In diesem Ausführungsbeispiel liegt der Rinneneinlauf 52 an der Spitze 40 des Gesichtsteils 30. Es ist auch denkbar, dass der Rinneneinlauf 52 an einem anderen Punkt des Gesichtsteils 30 liegt, beispielsweise an der Basis 45.

Der Rinneneinlauf 52 bildet durch eine sukzessive Vertiefung einen Übergang von der Außenseite 31 des Gesichtsteils 30 zu der endgültigen Vertiefung 59 der Führungsrinne 50.

Die Vertiefung 59 ist in der vorliegenden Ausführungsform nach dem Rinneneinlauf weitestgehend konstant tief ausgebildet. Die Vertiefung 59 kann auch unterschiedliche Tiefen aufweisen. Beispielsweise kann die Vertiefung 59 der Führungsrinne 50 an einem hierin weiter unten beschriebenen Endbereich 55 der Führungsrinne 50 tiefer ausgebildet sein.

Die Führungsrinne 50 umfasst einen hinteren Rinnenrand 51 und einen vorderen Rinnenrand 510. Der hintere Rinnenrand 51 ist derjenige Rinnenrand, der im Wesentlichen näher an der hinteren Ebene 400 liegt und der vordere Rinnenrand 510 ist derjenige Rinnenrand, der im Wesentlichen näher an der vorderen Ebene 500 liegt (siehe auch Fig. 5).

Im vorliegenden Ausführungsbeispiel haben der hintere Rinnenrand 51 und der vordere Rinnenrand 510 keinen Berührungspunkt. Es ist in anderen Ausführungen denkbar, dass sich der vordere Rinnenrand 510 und der hintere Rinnenrand 51 an einer oder mehreren Stellen verbinden, insbesondere dann, wenn die Außenseite 31 des Gesichtsteils 30 zwei oder mehrere Führungsrinnen 50 umfasst.

Die Rinnenränder 51, 510 sind im vorliegenden Ausführungsbeispiel durchgehend ausgebildet und weisen keine Unterbrechungen auf.

Die Rinnenränder 51, 510 weisen im vorliegenden Ausführungsbeispiel eine nahezu glatte Oberfläche auf. Es ist auch denkbar, dass einer der Rinnenränder 51, 510 und/oder beide Rinnenränder 51, 510 eine Struktur aufweisen, wie beispielsweise eine Riffelung.

Eine Struktur der Rinnenränder 51, 510 könnte die Führungseigenschaft verbessern, da der Anwender über die Riffelung eine taktile Rückmeldung über die Führung in der Führungsrinne 50 erhält. Beispielsweise kann die Riffelung auch eine andere gleichmäßig und ungleichmäßig ausgeführte Struktur sein. Die Struktur kann derart in den Rinnenrändern 51, 510 ausgebildet sein, dass der Anwender, der ein Verbindungselement 80 entlang eines Rinnenrandes 51, 510 der Führungsrinne 50 führt, eine taktile Rückmeldung über die Annäherung an das Aufnahmeelement 70 erhält. In diesem Beispiel wäre die Struktur des Rinnenrandes 51, 510 so angeordnet, dass die Struktur in den Bereichen des Rinnenrandes 51, 510, die näher am Aufnahmeelement 70 sind, zunehmend oder abnehmend gleichmäßiger oder ungleichmäßiger ist.

Die Führungsrinne weist eine Rinnenbreite 58 auf. Die Rinnenbreite 58 der Führungsrinne 50 steht im Wesentlichen in Abhängigkeit zur Lage des hinteren Rinnenrandes 51 zum vorderen Rinnenrand 510. Die Rinnenbreite 58 der Führungsrinne 50 kann konstant ausgebildet sein oder sich verändern.

Die Rinnenbreite 58 der Führungsrinne 50 ist am Rinneneinlauf 52 6 mm bis 12 mm, bevorzugt 8 mm bis 12 mm, beispielsweise 10 mm breit ausgebildet. Die Rinnenbreite 58 unmittelbar nach dem Rinneneinlauf 52 ist per definitionem Rinnenbreite 58A.

Eine Führungsrinne 50 mit einer konstanten Rinnenbreite 58, die der Rinnenbreite 58A entspricht, kann direkt vom Rinneneinlauf 52 zum Endbereich 55 (siehe unten) der Führungsrinne 50 verlaufen, der räumlich benachbart zu einem Aufnahmeelement 70 angeordnet ist.

Im in Fig. 8 und 9 dargestellten Ausführungsbeispiel weist die Führungsrinne 50 unterschiedliche Rinnenbreiten 58 auf. Die Führungsrinne 50 beginnt am Rinneneinlauf 52 mit einer Rinnenbreite 58A. Die Rinnenbreite 58A ist in einem ersten Abschnitt konstant. Ein erster Abschnitt der Führungsrinne 50 mit im Wesentlichen konstanter Rinnenbreite 58A wird hierin als Rinnenkanal 53 bezeichnet.

In einer Führungsrinne 50 mit konstanter Rinnenbreite 58 verlaufen der hintere Rinnenrand 51 und der vordere Rinnenrand 510 parallel zueinander. Ein paralleler Verlauf ist auch bei einem gebogenen Verlauf der Führungsrinne 50 mit konstanter Rinnenbreite 58 gegeben, die Rinnenränder 51, 510 beschreiben in dem Fall eine Parallelkurve.

Der Rinnenkanal 53 kann sich beliebig lang über die Länge des Gesichtsteils 30 erstrecken. Beispielsweise erstreckt sich der Rinnenkanal 53 über 50% der Länge des Gesichtsteils 30.

Im vorliegenden Ausführungsbeispiel nimmt die Rinnenbreite 58 der Führungsrinne 50 nach dem Rinnenkanal 53 zu. Als Rinnenbreite 58B wird diejenige Breite der Führungsrinne 50 bezeichnet, die eine größere Breite als Rinnenbreite 58A aufweist. Der Rinnenkanal 53 endet unmittelbar vor der Rinnenbreite 58B.

Ab der Rinnenbreite 58B kann die Führungsrinne 50 verbreitert ausgebildet sein. Beispielsweise kann die Rinnenbreite 58 sukzessive zunehmen. Die Führungsrinne weitet sich nach dem Rinnenkanal 53 ab der Rinnenbreite 58B bis zur maximalen Rinnenbreite 58C auf.

Im vorliegenden Ausführungsbeispiel nimmt die Rinnenbreite 58 der Führungsrinne nach der Rinnenbreite 58C wieder ab. Rinnenbreite 58D entspricht der Rinnenbreite 58B. Nach Erreichen der Rinnenbreite 58D verläuft die Führungsrinne 50 im vorliegenden Ausführungsbeispiel an der Basis 45 entlang und verbindet sich an der Symmetrieebene 300 mit dem Teil der Führungsrinne 50, der in identischer bzw. gespiegelter Form am linken oberen Ende beginnt und zur Basis 45 verläuft.

In dem abgebildeten Ausführungsbeispiel ist die Verbindung an der Basis 45 weniger breit ausgebildet als im oberen Teil des Führungskanals 50. An der Basis ist eine Rinnenbreite 58E, die eine geringere Breite aufweist als Rinnenbreiten 58A bzw. 58B und 58D.

Die Aufweitung der Rinnenbreite 58 zwischen der Rinnenbreite 58B und 58D geht mit einem Auseinanderlaufen des vorderen Rinnenrandes 510 und des hinteren Rinnenrandes 51 einher. Der vordere Rinnenrand 510 führt ab der Rinnenbreite 58B im Wesentlichen senkrecht am Auslass 36 entlang und verläuft nahe bei der Symmetrieebene 300.

Der hintere Rinnenrand 51 entfernt sich ab der Rinnenbreite 58B von der Symmetrieebene 300 und verläuft in Richtung der hinteren Ebene 400, so dass sich die Führungsrinne 50 in der horizontalen Ebene 200 aufweitet. Die Rinnenbreite 58C ist die breiteste Stelle der Führungsrinne 50 in horizontaler Richtung. Ab der Rinnenbreite 58C verläuft der hintere Rinnenrand 51 wieder in Richtung der Symmetrieebene 300.

Der horizontale Verlauf der Führungsrinne 50 zwischen der Rinnenbreite 58B und der Rinnenbreite 58D ist buchtförmig und wird als Rinnenbucht 54 bezeichnet. Buchtförmig bedeutet definitionsgemäß, dass durch ein bogenartiges Zurückweichen des hinteren Rinnenrands 51 die Führungsrinne 50 in diesem Bereich die Form einer Bucht oder eines offenen Bogens aufweist. Die Rinnenbucht 54 kann beispielsweise einen runden Verlauf aufweisen, ein eckiger Verlauf ist auch denkbar.Die Rinnenbucht 54 wird vom hinteren Rinnenrand 51 umschlossen.

Im dargestellten Ausführungsbeispiel endet die Führungsrinne 50 in der Horizontalen Ebene 200 an einem Endbereich 55. Der Endbereich 55 ist in horizontaler Richtung maximal entfernt von der Symmetrieebene 300 (Fig. 5) angeordnet.

Der Endbereich 55 liegt auf einer Ebene mit einem hierin weiter unten beschriebenen Rinnenboden 56 und liegt räumlich benachbart zu einem Aufnahmeelement 70, das die Führungsrinne 50 in diesem Endbereich 55 überspannt. Der Endbereich 55 wird im vorliegenden Ausführungsbeispiel vom hinteren Rinnenrand 51 buchtförmig umschlossen.

Der Endbereich 55 kann tiefer ausgeprägt sein als der Rest der Führungsrinne 50. Die Vertiefung 59 im Endbereich 55 ist zwischen 1 mm und 6 mm tief ausgebildet. Beispielsweise ist die Vertiefung 59 im Bereich des Endbereichs 4 mm tief.

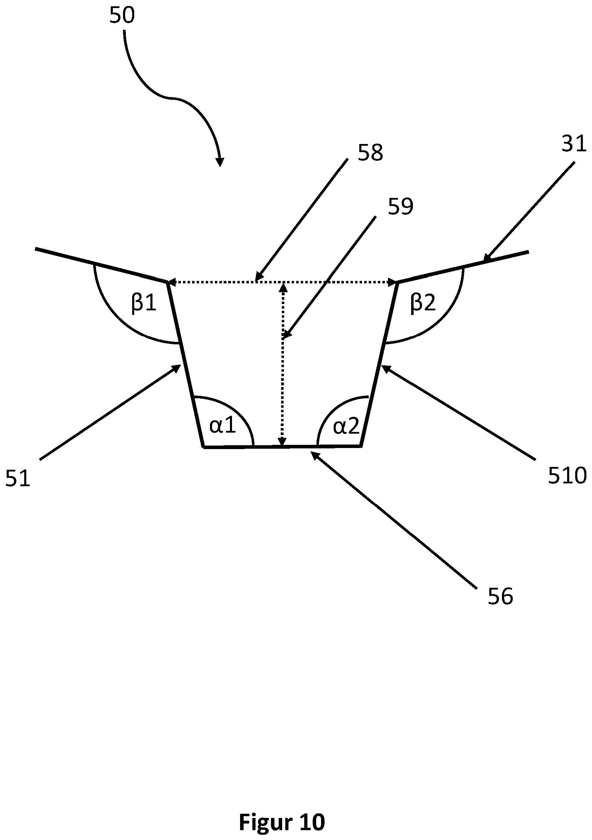

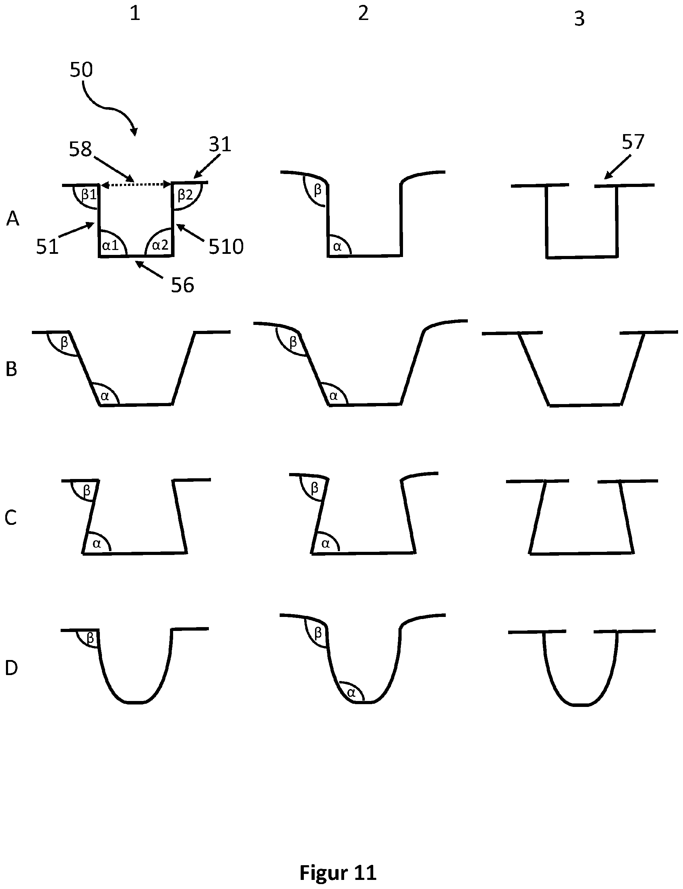

In **Fig. 10** **und** **11** sind beispielhaft schematische Abbildungen einer Führungsrinne 50 im Querschnitt gezeigt. Die Führungsrinne 50 umfasst einen Rinnenboden 56, einen hinteren Rinnenrand 51, einen vorderen Rinnenrand 510 und eine Rinnenbreite 58.

Die Führungsrinne 50 ist begrenzt durch den Rinnenboden 56. Der Rinnenboden 56 weist im vorliegenden Ausführungsbeispiel eine nahezu glatte Oberfläche auf. Es ist auch denkbar, dass der Rinnenboden 56 eine Struktur aufweist, wie beispielsweise eine Riffelung. Eine Struktur könnte die Führungseigenschaft verbessern, da der Anwender über die Riffelung eine taktile Rückmeldung über die Führung in der Führungsrinne 50 erhält. Beispielsweise kann die Riffelung auch eine andere gleichmäßig und ungleichmäßig ausgeführte Struktur sein. Die Struktur kann derart in der Führungsrinne 50 ausgebildet sein, dass der Anwender, der ein Verbindungselement 80 entlang der Führungsrinne 50 führt, eine taktile Rückmeldung über die Annäherung an das Aufnahmeelement 70 erhält. In diesem Beispiel wäre die Struktur in der Führungsrinne 50 so angeordnet, dass die Struktur in den Bereichen der Führungsrinne 50, die näher an dem Aufnahmeelement 70 sind, zunehmend oder abnehmend gleichmäßiger oder ungleichmäßiger ist.

Die Führungsrinne 50 ist seitlich begrenzt durch den hinteren Rinnenrand 51 und den vorderen Rinnenrand 510. Wie aus Fig. 10/11 ersichtlich, kann der Rinnenrand 51, 510 von der Außenseite 31 zum Rinnenboden 56 beispielsweise gerade verlaufen. Eine konvexe oder konkave Wölbung des Rinnenrandes 51 und/oder 510 ist auch denkbar.

Ein Winkel α1 und ein Winkel α2 beschreiben, in welchem Winkel der hintere Rinnenrand 51 und der vordere Rinnenrand 510 auf dem Rinnenboden 56 angeordnet ist. Die Winkel α1 und Winkel α2 können konstant sein. Die Winkel α1 und/ oder Winkel α2 können im Verlauf der Führungsrinne unterschiedliche Werte aufweisen. Die Winkel α1 und Winkel α2 sind unabhängig voneinander ausgebildet.

Der Rinnenrand 51, 510 kann mit einem Winkel α1, α2 von 90° senkrecht auf dem Rinnenboden 56 angeordnet sein (Fig. 11 A).

Bevorzugt ist der Rinnenrand 51, 510 geneigt, beispielsweise nach außen geneigt (Fig. 10 und 11 B). Beispielsweise kann der Rinnenrand 51, 510 mit einem stumpfen Winkel α1, α2 auf dem Rinnenboden angeordnet sein. Der Winkel α1, α2 kann 90° bis 180°, bevorzugt 90° bis 135° betragen. In vorteilhaften Ausführungsform kann der Winkel α2 beispielsweise zwischen 90 und 95° betragen und der Winkel α1 variierend zwischen 90 und 135° ausgebildet sein.

Ein Winkel α1, α2 > 90° ist geeignet, wenn das Gesichtsteil 30 im Spritzgußverfahren hergestellt wird, da sich das Gesichtsteil 30 sodann leicht von der Form lösen lässt. Ferner ist die Reinigung einer nach außen geneigten Führungsrinne 50 erleichtert.

Der Winkel α1, α2 kann auch kleiner als 90° sein, so dass der Rinnenrand 51, 510 nach innen geneigt angeordnet ist (siehe Fig. 11 C).

Eine Ausführungsform mit einem Winkel α1, α2 < 90° könnte vorteilhaft sein, da ein nach innen geneigter Rinnenrand 51, 510 bessere und sicherere Führungseigenschaften für das Verbindungselement 80 bieten kann.

Besonders vorteilhaft für die Anwendung könnte beispielsweise auch ein nach innen überragender Überhang 57 am Rinnenrand 51 und/oder am Rinnenrand 510 sein (siehe Fig. 11 A-D, Reihe 3). Ein nach innen überragender Überhang 57 könnte die Führungseigenschaft der Führungsrinne 50 verbessern, da Bauteile des Verbindungselements 80, wie beispielsweise die hierin unten beschriebene Knopfplatte 91, zwischen Überhang 57 und Rinnenboden 56 entlanggeführt werden können, was die Führungssicherheit erhöhen kann.

Der Übergang vom Rinnenboden 56 zum Rinnenrand 51, 510 kann eckig (Fig. 11 A-C) oder abgerundet (Fig. 11 D) sein. Ein gerundeter Übergang kann vorteilhaft bezüglich einer Reinigung sein.

Ein Winkel β1 und Winkel β2 beschreiben, in welchem Winkel der hintere Rinnenrand 51 und der vordere Rinnenrand 510 zu der Außenseite 31 angeordnet sind. Die Winkel β1 und β2 können beispielsweise konstant sein. Es ist auch denkbar, dass der Winkel β1 und/ oder der Winkel β2 im Verlauf der Führungsrinne unterschiedliche Werte aufweisen. Die Winkel β1 und Winkel β2 sind unabhängig voneinander ausgebildet.

Der Rinnenrand 51, 510 kann mit einem Winkel β1, β2 von 90° senkrecht von der Außenseite 31 abfallen (Fig. 11 A).

Im vorliegenden Ausführungsbeispiel ist der Winkel β1, β2 größer als 90° (Fig. 10 und 11 B + D). Beispielsweise kann der Rinnenrand 51, 510 mit einem stumpfen Winkel β1, β2 zu der Außenseite 31 angeordnet sein. Der Winkel β1, β2 kann 90° bis 180°, bevorzugt 90° bis 135° betragen. In vorteilhaften Ausführungsform kann der Winkel β2 beispielsweise zwischen 90 und 100° betragen und der Winkel β1 variierend zwischen 90 und 135° ausgebildet sein.

Es ist auch denkbar, dass der Rinnenrand 51, 510 mit einem spitzen Winkel β1, β2 von beispielsweise 45 bis 90 ° zu der Außenseite 31 angeordnet ist (Fig. 11 C).

Der Übergang von der Außenseite 31 zum Rinnenrand 51, 510 kann eckig (siehe Fig. 11, Reihe 1 + 3) oder abgerundet (siehe Fig. 11, Reihe 2) sein. Ein gerundeter Übergang kann vorteilhaft bezüglich einer Reinigung sein.

Die Rinnenbreite 58 der Führungsrinne 50 steht in Abhängigkeit zur Ausrichtung des Rinnenrandes 51, 510 und den Winkeln α1, α2 und β1, β2 sowie zur Breite des Rinnenbodens 56. Die Rinnenbreite 58 ist im vorliegenden Ausführungsbeispiel breiter als der Rinnenboden 56 (Fig. 10 und 11 B). Die Rinnenbreite 58 kann auch schmaler (Fig. 11 C) oder gleich breit (Fig. 11 A) sein wie der Rinnenboden 56. Das Verhältnis der Rinnenbreite 58 zur Breite des Rinnenbodens 56 kann beispielsweise konstant sein. Das Verhältnis kann sich im Verlauf der Führungsrinne 50 jedoch auch verändern.

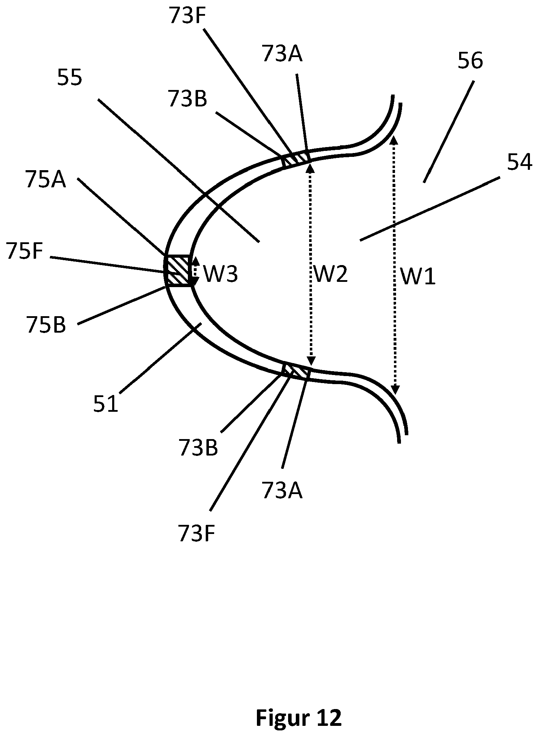

**Fig. 12** zeigt in einer Draufsicht eine schematische Übersicht des hinteren Rinnenrandes 51 im Bereich der Rinnenbucht 54. Das diesen Bereich überspannende, hierin nachfolgend beschriebene Aufnahmeelement 70 ist nicht abgebildet.

Der Rinnenboden 56 der Rinnenbucht 54 weist unterschiedliche Weiten auf. Der Rinnenboden 56 der Rinnenbucht 54 nimmt in der Horizontalen in der Weite ab. Das bedeutet, dass eine erste Rinnenbucht-Weite W1 größer als eine zweite Rinnenbucht-Weite W2 ist und die Rinnenbucht-Weite W2 größer als eine dritte Rinnenbucht-Weite W3 ist.

Die Rinnenbucht-Weite W1 ist die Weite des Rinnenbodens 56, der durch den horizontalen Verlauf des hinteren Rinnenrandes 51 zwischen der Rinnenbreite 58B und der Rinnenbreite 58D entsteht. Die Rinnenbucht-Weite W2 ist definitionsgemäß die Weite des Rinnenbodens 56 zwischen zwei vorderen Steg-Ansätzen 73A (hierin weiter unten beschrieben). Die Rinnenbucht-Weite W3 ist definitionsgemäß die Weite des Rinnenbodens 56 zwischen einem oberen Mittelsteg-Ansatz 75A und einem unteren Mittelsteg-Ansatz 75B (hierin weiter unten beschrieben).

Der Endbereich 55 der Führungsrinne 50 liegt am horizontalen Ende der Führungsrinne 50 in der Rinnenbucht 54 zwischen einer Rinnenbucht-Weite W2 und einer Rinnenbucht-Weite W3.

Die Höhe der hinteren Rinnenwand 51 und somit die Vertiefung 59 der Führungsrinne 50 kann im Bereich der Rinnenbucht 54 konstant sein. Vorteilhaft - und in diesem Ausführungsbeispiel dargestellt - ist, dass die hintere Rinnenwand 51 im Bereich der Rinnenbucht 54 in der Höhe zunimmt. Die Höhe des hinteren Rinnenrandes 51 kann im Verlauf der Rinnenbucht 54 von der Weite 1 zur Weite 3 sukzessive zunehmen.

Durch den erhöhten Rinnenrand 51 im Bereich der Rinnenbucht 54 und somit auch im Endbereich 55 nimmt die dreidimensionale Ausdehnung der Führungsrinne 50 in diesem Endbereich 55 zu.

Die Führungsrinne 50 und insbesondere die Rinnenränder 51, 510 ermöglicht dem Anwender und/oder Patienten, zu dem Endbereich 55 zu gelangen. Der Anwender und/oder Patienten kann den Endbereich 55 beispielsweise durch Ertasten erreichen.

Es ist im speziellen daran gedacht, dass der Anwender und/oder der Patient mit den Fingern oder insbesondere mit dem hierin nachfolgend beschriebenen Verbindungselement 80 an dem Rinnenrand 51 und/oder 510 entlangfahren kann und so den Endbereich 55 erreicht.

Im vorliegenden Ausführungsbeispiel ist der horizontale Verlauf der Führungsrinne 50 buchtförmig 54. Dadurch wird es dem Anwender und/oder dem Patienten ermöglicht, zu einem Endbereich 55, der in der Rinnenbucht 54 liegt, zu gelangen, indem er an sich an dem hinteren Rinnenrand 51 orientiert.

Beispielsweise kann der Anwender und/oder der Patient ein hierin nachfolgend beschriebenes Verbindungselement 80 entlang des hinteren Rinnenrandes 51 zum Endbereich 55 führen, wo dieses wie nachfolgend beschrieben durch Aufnahme in einem Aufnahmeelement 70 an der Atemmaske 100 befestigt werden kann.

Wie aus Fig. 4 ersichtlich wird, sind in diesem Ausführungsbeispiel an den beiden unteren Eckpunkten 43/44 des Gesichtsteils 30 zwei Ankerpunkte 60/61 angeordnet. Es ist auch denkbar, dass weitere Ankerpunkte am Gesichtsteil 30 angeordnet sind. Weitere Ankerpunkte könnten auch abseits der unteren Eckpunkte 43/44 angeordnet sein, beispielweise weiter oben 310 liegend.

Die Ankerpunkte 60/61 sind beidseitig der Symmetrieebene 300 angeordnet und stellen dementsprechend und wie weiter oben dargestellt einen linken Ankerpunkt 60 und einen rechten Ankerpunkt 61 dar. Die Ankerpunkte 60/61 sind bevorzugt maximal weit von der Symmetrieebene 300 entfernt.

Aus Fig. 5 wird ersichtlich, dass die beispielsweise zwei Ankerpunkte 60/61 zwischen der mittleren Ebene 450 und der hinteren Ebene 400 angeordnet sind. Die Ankerpunkte 60/61 umfassen jeweils ein Aufnahmeelement 70 und ein Verbindungselement 80.

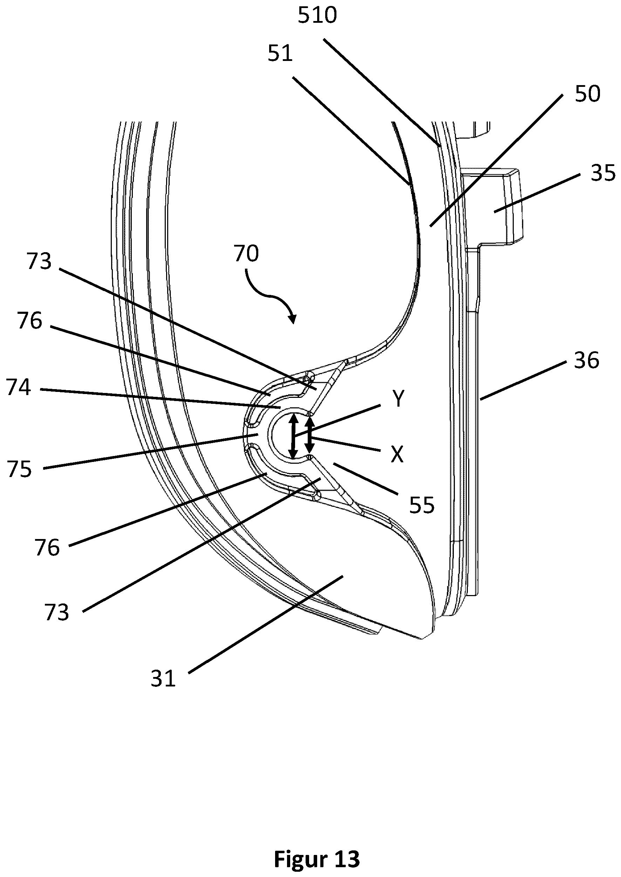

Die Außenseite 31 umfasst an den Ankerpunkten 60/61 jeweils ein Aufnahmeelement 70. Fig. 13 bis 16 zeigen ein Ausführungsbeispiel für ein Aufnahmeelement 70.

**Fig. 13** zeigt das Aufnahmeelement 70 in einer Übersicht in Draufsicht. Das Aufnahmeelement 70 ist ein integraler Bestandteil der Außenseite 31 und des hinteren Rinnenrandes 51.

Das Aufnahmeelement 70 ist vorteilhafterweise aus einem Material gefertigt, dass auf der einen Seite eine gewisse Nachgiebigkeit und eine geringe Spannungsrissempfindlichkeit aufweist und auf der anderen Seite eine ausreichende Stabilität. Das Aufnahmeelement 70 ist beispielsweise einteilig und aus demselben Material wie das Gesichtsteil 30 gefertigt. Eine zweiteilige Ausführung ist auch denkbar. Das Aufnahmeelement 70 ist beispielsweise aus Polyamid PA12 gefertigt. Das Polyamid PA12 ist sehr stabil, weist eine geringe Spannungsrissempfindlichkeit auf und ist temperaturbeständig.

Das Aufnahmeelement 70 ist zwischen der Weite W2 und der Weite W3 der Rinnenbucht 54 angeordnet (siehe Fig. 12) und überspannt den Endbereich 55 der Führungsrinne 50.

Das Aufnahmeelement 70 umfasst im vorliegenden Ausführungsbeispiel zwei Stege 73, eine Brücke 74, einen Aufnahme-Durchmesser Y und eine Aufnahme-Öffnung X. Das Aufnahmeelement kann zudem beispielsweise mindestens einen Mittelsteg 75 und mindestens einen, bevorzugt 2 Durchbrüche 76 umfassen.

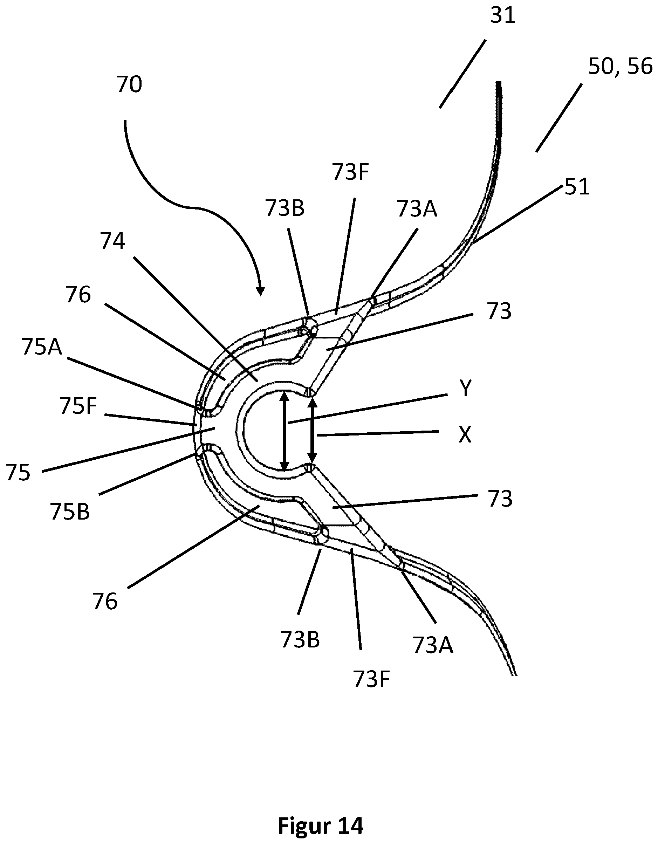

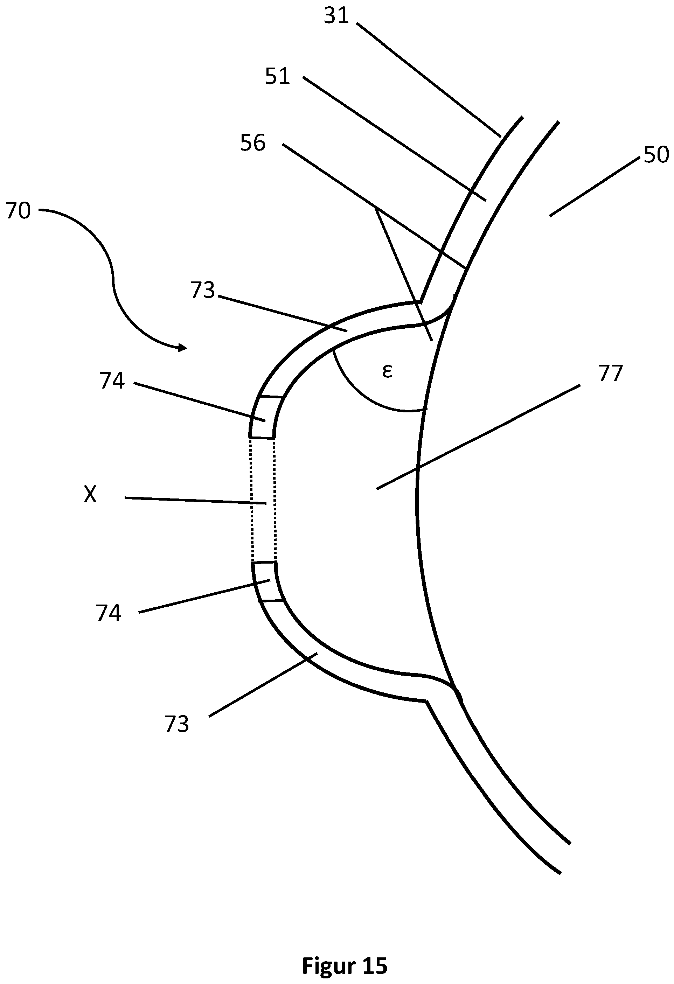

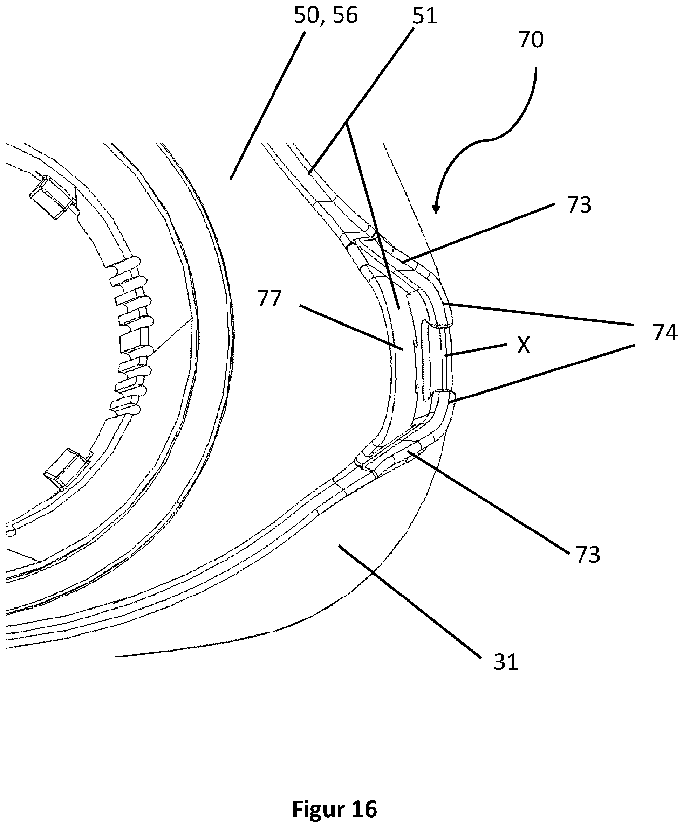

**Fig. 14** zeigt das Aufnahmeelement 70 in einer detaillierteren Draufsicht. **Fig. 15** zeigt eine schematische Abbildung des Aufnahmeelements 70 von vorne 520. **Fig. 16** zeigt einen Ausschnitt eines Gesichtsteils 30 von vorne 520 zur Darstellung eines Aufnahmeelements 70 von vorne 520.

Die Stege 73 gehen aus dem hinteren Rinnenrand 51 hervor. Die Stege 73 erheben sich über den Rinnenboden 56 der Führungsrinne 50 und haben im Wesentlichen keinen Kontakt zum Rinnenboden 56 (siehe Fig. 15). Die Stege sind über Steg-Ansatzflächen 73F mit dem hinteren Rinnenrand 51 verbunden.

Die Steg-Ansatzflächen 73F sind zwischen 3 mm und 6 mm, beispielsweise 5 mm breit und erstrecken sich von einem vorderen Steg-Ansatz 73A zu einem hinteren Steg-Ansatz 73B.

Die Breite der Stege 73 kann an der Steg-Ansatzfläche 73F breiter ausgebildet sein (beispielsweise 5 mm). Die Breite der Stege 73 kann im weiteren Verlauf in der Breite abnehmen auf einen Bereich 4 mm bis 1 mm. Die Stege sind im Wesentlichen beispielsweise 3,3 mm breit.

Die Stege 73 können beispielsweise eine konstante Materialstärke aufweisen. Die Stege 73 sind zwischen 1 mm und 4 mm, beispielsweise 2 mm dick. Die Stege 73 können 2 mm bis 20 mm, beispielsweise 14 mm lang sein. Die Stege 73 sind in Breite, Länge und Dicke derart aufeinander abgestimmt ausgebildet, dass eine ausreichende Nachgiebigkeit gegeben ist.

Die Stege 73 können beispielsweise geschwungen oder gebogen verlaufen. Das bedeutet, dass die Stege nicht gerade verlaufen müssen.

Ein Winkel ε beschreibt, in welchem Winkel die Stege 73 zum Rinnenboden 56 angeordnet sind (siehe Fig. 15). Die Stege 73 können mit einem Winkel ε von 90° senkrecht zum Rinnenboden 56 angeordnet sein. Bevorzugt sind die Stege 73 mit einem Winkel ε von 30° bis 90°, beispielsweise mit einem Winkel ε von 30° bis 60° zum Rinnenboden 56 angeordnet.

Benachbart zu den Stegen 73 ist eine Brücke 74 angeordnet. Brücke 74 und Stege 73 sind beispielsweise einstückig ausgebildet.

In diesem Ausführungsbeispiel ist die Brücke 74 in Breite und Dicke äquivalent zu den Stegen 73 ausgebildet, wohingegen die Brücke 74 länger als die Stege 73 ausgebildet sein kann.

Die Brücke 74 ist 1 mm bis 4 mm, beispielsweise 2 mm dick. Die Brücke 74 kann 10 mm bis 30 mm, beispielsweise 14 mm lang sein. Die Brücke 74 ist 1 mm bis 4 mm, beispielsweise 2,3 mm breit.

Die Brücke 74 ist beispielsweise kreisrund ausgebildet. Die Brücke 74 ist nicht geschlossen kreisrund ausgebildet ist, sondern ist unterbrochen durch eine Aufnahme-Öffnung X. Die Aufnahme-Öffnung X ist zwischen 3 mm und 12 mm, bevorzugt zwischen 5 mm und 8 mm, beispielsweise 5,8 mm weit ausgebildet.

Die Aufnahme-Öffnung X ist nach vorne 520 ausgerichtet. Durch die Aufnahme-Öffnung X ist die Brücke 74 annähernd halbkreisförmig ausgebildet. Die Aufnahme-Öffnung X ist ausgebildet, einen hierin nachfolgend beschriebenen Hals 93 des Verbindungselements 80 aufzunehmen.

Die Aufnahme-Öffnung X ist etwas kleiner ausgebildet als der Durchmesser des Halses 93D, so dass die Aufnahme des Halses nur über Anwendung von Druck und eine leichte Nachgiebigkeit des Aufnahmeelements 70 möglich ist.

Die Brücke 74 weist einen Aufnahme-Durchmesser Y auf. Der Aufnahme-Durchmesser Y der Brücke 74 beträgt zwischen 3 mm und 12 mm, bevorzugt zwischen 5 mm und 8 mm, beispielsweise 6,2 mm.

Der Aufnahme-Durchmesser Y ist etwas größer ausgebildet als die Aufnahme-Öffnung X. Der Aufnahme-Durchmesser Y ist ausgebildet, eine hierin nachfolgend beschriebenen Hals 91 des Verbindungselements 80 aufzunehmen. Der Aufnahme-Durchmesser Y ist etwas größer ausgebildet als der Durchmesser des Halses 93D.

Dadurch, dass die Stege 73 gebogen ausgebildet sind und sich mit dem Winkel ε über den Rinnenboden 56 erheben, entsteht der Aufnahmeraum 77 zwischen Stegen 73, Brücke 74 und dem Rinnenboden 56 (siehe Fig. 15 und Fig. 16).

Der Aufnahmeraum 77 befindet sich am Endbereich 55 (siehe Fig. 13). Der Aufnahmeraum 77 weist eine Höhe von 2 mm bis 8 mm, beispielsweise 4 mm auf. Der Aufnahmeraum 77 ist ausgebildet, eine hierin nachfolgend beschriebenen Knopfplatte 91 inklusive Überhang 92 des Verbindungselements 80 aufzunehmen. Die Tiefe des Aufnahmeraums 77 ist demnach größer ausgebildet als die Länge der Knopfplatte 91L.

Der mindestens eine Mittelsteg 75 verbindet die Brücke 74 mit der Außenseite 31. Der Mittelsteg 75 befindet sich beim vorliegenden Ausführungsbeispiel auf der Hälfte der halbkreisförmigen Brücke 74 (Fig. 13).

Aus Fig. 14 wird ersichtlich, dass der Mittelsteg 75 über eine Mittelsteg-Ansatzfläche 75F mit dem hinteren Rinnenrand 51 verbunden ist. Die Mittelsteg-Ansatzfläche 75F ist zwischen 1 und 6 mm, beispielsweise 3 mm breit und erstrecken sich von einem oberen Mittelsteg-Ansatz 75A zu einem unteren Mittelsteg-Ansatz 75B. Die Breite des Mittelstegs 75 kann an der Mittelsteg-Ansatzfläche 75F breiter ausgebildet sein, beispielsweise 3 mm, und im weiteren Verlauf in der Breite abnehmen auf beispielsweise 2 mm.

Die Breite der Mittelsteg-Ansatzfläche 75F vom oberen Mittelsteg-Ansatz 75A zu einem unteren Mittelsteg-Ansatz 75B entspricht der oben beschriebenen Weite W3 des Rinnentrichters (siehe Fig. 12).

Der mindestens eine Durchbruch 76, in diesem Ausführungsbeispiel die 2 Durchbrüche 76 sind zwischen dem Mittelsteg 75 und den Stegen 73 angeordnet. Die Durchbrüche 76 ermöglichen eine leichtere Bauweise und Materialersparnis. Zudem kann, abhängig vom gewählten Material, eine Nachgiebigkeit erreicht werden, die ein leichtes Einrasten und Lösen des Verbindungselementes 80 gewährleistet.

Es ist auch denkbar, dass das Aufnahmeelement 70 ohne Durchbrüche 76 und Mittelsteg 75 ausgebildet ist. In dem Falle würde die hintere Rinnenwand 51 und die Außenseite 31 direkt in die Brücke 74 und Stege 73 übergehen (nicht abgebildet).

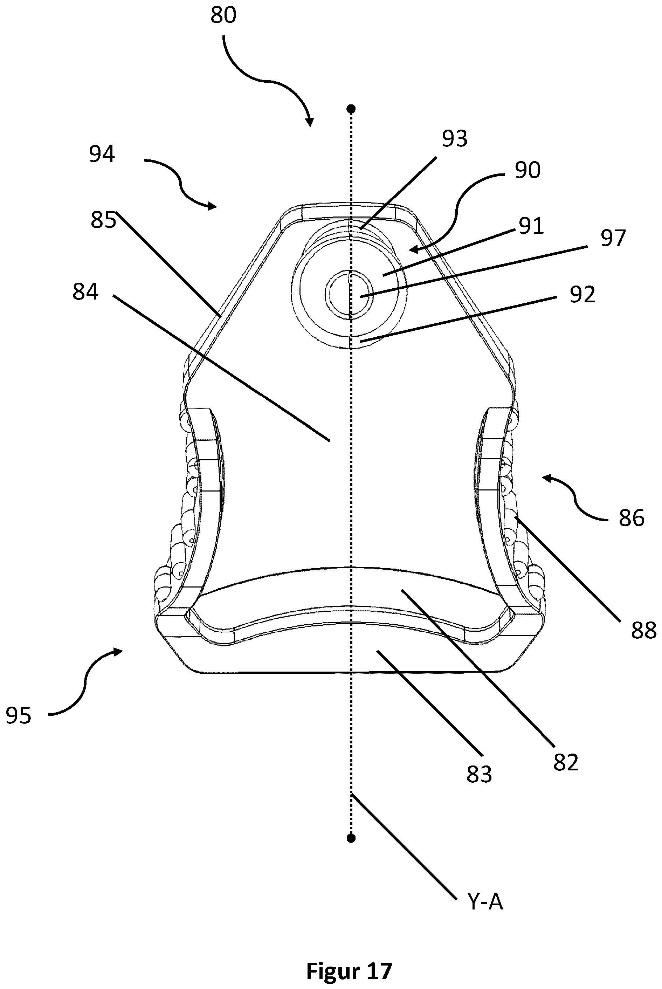

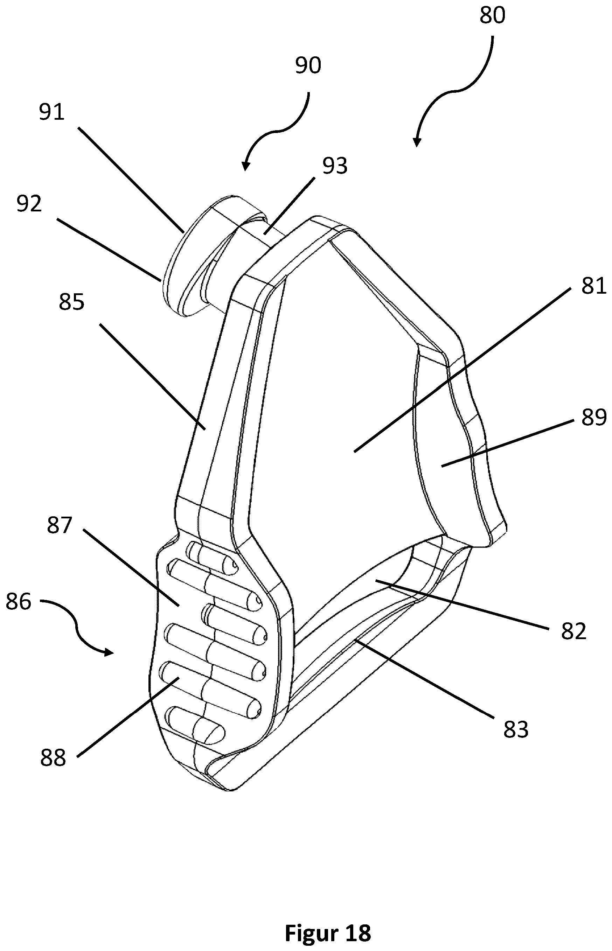

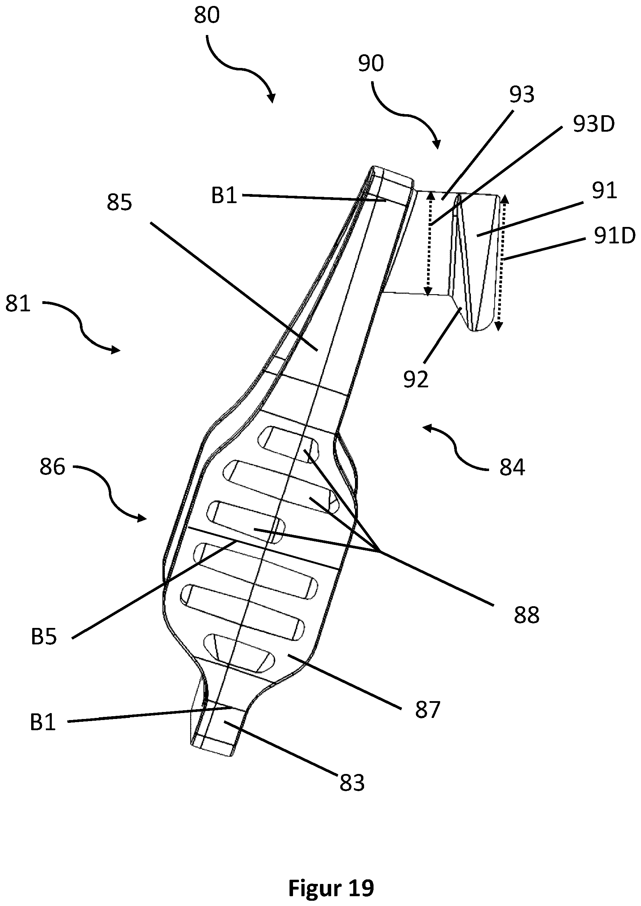

Die Figuren 17 bis 19 zeigen ein Ausführungsbeispiel für ein erfindungsgemäßes Verbindungselement 80. Fig. 17 zeigt ein Verbindungselement 80 in einer Draufsicht von hinten 420. Fig. 18 zeigt ein Verbindungselement 80 in einer perspektivischen Ansicht schräg von vorne 520. Fig. 19 ein Verbindungselement 80 von der Seite.

Das Verbindungselement 80 ist ein lösbares Bauteil der Atemmaske 100. Das Verbindungselement 80 kann aus einem beliebigen Werkstoff gefertigt sein, das eine ausreichende Stabilität aufweist. Das Verbindungselement 80 ist bevorzugt aus demselben Material wie das Gesichtsteil 30 gefertigt. Das Verbindungselement 80 ist bevorzugt aus einem harten Kunststoff gefertigt. Geeignete Kunststoffe umfassen beispielsweise Polyamide, Polycarbonate, Polyoxymethylene, Polysulfone und Polypropylene. Beispielsweise ist das Verbindungselement 80 aus Polyamid PA12 gefertigt. Das Verbindungselement 80 ist beispielsweise vollständig aus Polyamid PA12 gefertigt. Es ist auch denkbar, dass unterschiedliche Bauteile des Verbindungselements 80 aus unterschiedlichen Materialien bestehen.

**Fig. 17** zeigt beispielhaft eine Ausführungsform des Verbindungselements 80 in einer Draufsicht von hinten 420. In dieser Ansicht ist diejenige Fläche abgebildet, die nach Befestigung am Aufnahmeelement 70 dem Gesichtsteil 30 (nicht gezeigt) zugewandt ist. Definitionsgemäß ist diese Fläche die innere Grundfläche 84 des Verbindungselements 80. In dieser Ausführungsform hat das Verbindungselement 80 eine sechseckige Grundfläche. Die Grundfläche kann auch jede andere geeignete Form aufweisen.

Die Grundfläche ist plan ausgebildet. Die Grundfläche des Verbindungselements 80 ist somit in dieser Ausführungsform nicht gekrümmt oder gewölbt. Es ist aber auch denkbar, dass die Grundfläche des Verbindungselements 80 gekrümmt oder gewölbt ausgebildet ist.

Das Verbindungselement 80 umfasst ein Befestigungselement 90. Per definitionem befindet sich das Befestigungselement 90 am oberen Ende 94 des Verbindungselements 80. Das Verbindungselement 80 umfasst zudem ein Haltesteg 83 und eine Aussparung 82. Per definitionem befinden sich Haltesteg 83 und Aussparung 82 am unteren Ende 95 des Verbindungselements 80. Somit liegen Befestigungselement 90 und Haltesteg 83 an sich gegenüberliegenden Seiten des Verbindungselements.

Das Verbindungselement 80 kann über das Befestigungselement 90 an dem Gesichtsteil 30, genauer gesagt an dem Aufnahmeelement 70 des Gesichtsteils 30, manuell befestigt werden. Die Befestigung ist reversibel, das heißt, die Befestigung kann jederzeit manuell hergestellt und wieder gelöst werden, ohne dass das Material eine bleibende Veränderung erfährt.

Durch die Aussparung 82 kann eine nicht abgebildete Bänderung 96 verlaufen. Die Bänderung 96 kann über den Haltesteg 83 verlaufen und dient zur Befestigung der Atemmaske 100 am nicht gezeigten Kopf eines Anwenders und / oder Patienten.

Das Verbindungselement 80 ist in dieser Ausführungsform achsensymmetrisch um eine y-Achse ausgebildet.

Das Verbindungselement 80 hat eine Länge in einem Bereich von 100 mm bis 10 mm, bevorzugt von 50 mm bis 20 mm, beispielsweise 35 mm. Das Verbindungselement 80 hat eine maximale Breite von 80 mm bis 10 mm, bevorzugt von 40 bis 20 mm, beispielsweise 27 cm. Die Grundfläche des Verbindungselements 80 hat eine Dicke von 1 mm bis 3 mm, beispielsweise 2 mm.

**Fig. 18** zeigt ein Verbindungselement 80 in einer perspektivischen Ansicht schräg von vorne 520. Fig. 18 ist zu entnehmen, dass das Verbindungselement 80 eine umlaufende Seitenwand 85 mit mindestens einer, bevorzugt zwei Griffmulden 86 umfasst. Die Griffmulden 86 umfassen mindestens ein Strukturelement 88.

Das Verbindungselement 80 hat eine Gesamtlänge von beispielsweise 35 mm (siehe oben). Die Griffmulden 86 haben eine Länge von 5 mm bis 30 mm, bevorzugt von 10 bis 20 mm, beispielsweise 17 mm. In anderen Worten erstreckt sich die Griffmulde 86 bevorzugt über etwa 50 % der Länge des Verbindungselements 80.

**Fig. 19** zeigt ein Verbindungselement 80 von der Seite. Aus Fig. 19 wird ersichtlich, dass die Seitenwand 85 unterschiedlich breit ausgebildet sein kann. Die Breite der Seitenwand 85 liegt in einem Bereich von 20 mm bis 1 mm, bevorzugt von 15 mm bis 1 mm. In diesem Ausführungsbeispiel ist die Seitenwand 85 am oberen Ende 94 ca. 2 mm breit **(B1)** und nimmt in der Breite zu.

Die Seitenwand 85 weist auf Höhe der Griffmulden 86 die größte Breite von ca. 10 mm **(B5)** auf. Nach den Griffmulden 86 verjüngt sich die Seitenwand 85 wieder auf ihre Anfangsbreite **(B1).** Somit ist die Griffmulde 86 in diesem Ausführungsbeispiel ca. 5-mal so breit wie die Seitenwand 85 am oberen Ende 94 bzw. am unteren Ende 95 des Verbindungselements 80.

Die Griffmulden 86 dienen dazu, das Verbindungelement 80 mit zwei Fingern, beispielsweise mit Zeigefinger und Daumen zu ergreifen. Die Länge und Breite der Griffmulde 86 ist vorzugsweise derart gewählt, dass der Anwender und/oder der Patient das Verbindungselement 80 gut und sicher mit zwei Fingern ergreifen kann.

Die Griffmulden-Außenwand 87, also die Seitenwand 85 in Höhe der Griffmulden 86, weist mindestens ein, bevorzugt mehrere, beispielsweise 6 Strukturelemente 88 auf. Die Strukturelemente 88 dienen dazu, die Griffigkeit der Griffmulden 86 zu erhöhen.

Das mindestens eine Strukturelement 88 umfasst beispielsweise mindestens eine Erhöhung z.B. in Form von Rippen, Wellen, Gitterstrukturen, Netzen, Punkten o.ä. In der vorliegenden Ausführungsform sind die Strukturelemente 88 in Form von rippenartigen Erhöhungen ausgebildet, die auf der Griffmulden-Außenwand 87 angeordnet sind.

Über die Strukturelemente 88 der Griffmulden-Außenwand 87 wird die Griffigkeit der Griffmulden 86 erhöht. Die Form der Strukturelemente 88 ist derart gewählt, dass sie in Bezug auf Griffigkeit, Komfort und Stabilität optimal ausgebildet sind.

Die Griffmulden-Innenwand 89 ist hingegen bevorzugt glatt ausgebildet (siehe Fig. 18). Eine glatte Ausbildung der Griffmulden-Innenwand 89 ist vorteilhaft, da derart Material und somit Gewicht eingespart werden kann und die Griffmulden-Innenwand 89 einfacher und effizienter gereinigt werden kann.

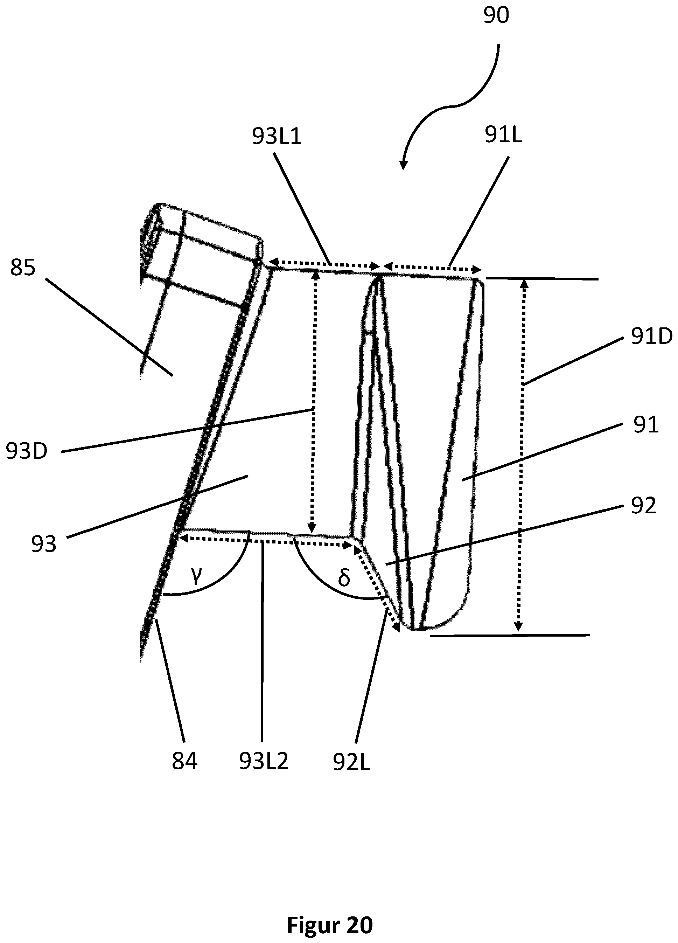

Das Verbindungselements 80 umfasst ein Befestigungselement 90. **Fig. 20** zeigt das Befestigungselement 90 im Detail. Verbindungselement 80 und Befestigungselement 90 können einoder 2-teilig ausgebildet sein. In diesem Ausführungsbeispiel sind Verbindungselement 80 und Befestigungselement 90 einteilig ausgebildet. Das Befestigungselement 90 ist ein Bestandteil des Verbindungselements 80 und Verbindungselement 80 und Befestigungselement 90 bestehen aus demselben Material.

Das Befestigungselement 90 umfasst eine Knopfplatte 91, einen Überhang 92 und einen Hals 93. Die Knopfplatte 91 ist über den Hals 93 mit der inneren Grundfläche 84 des Verbindungselements 80 verbunden.

Der Hals 93 ist rund oder gerundet ausgeführt und weist einen maximalen Durchmesser 93D von 3 mm bis 12 mm, beispielsweise 6 mm auf. Der Durchmesser 93D ist so ausgeführt, dass der Hals 93 nur mit leichtem Druck durch die Aufnahme-Öffnung X (Fig. 13) geführt werden kann. Der Durchmesser 93D ist so ausgeführt, dass er etwas größer ist als die Aufnahme-Öffnung X. Der Durchmesser 93D ist so ausgeführt, dass er etwas kleiner ist als der Aufnahme-Durchmesser Y (Fig. 13).

Die Knopfplatte 91 ist rund oder gerundet ausgeführt und weist einen maximalen Durchmesser 91D von 3 mm bis 14 mm, beispielsweise 8 mm auf. Der Durchmesser der Knopfplatte 91D ist größer als der Durchmesser des Halses 93D.

Bei Hindurchführung des Halses 93 durch die Aufnahme-Öffnung X in den Aufnahme-Durchmesser Y gelangt die Knopfplatte 91 in den Aufnahmeraum 77. Die Knopfplatte 91 dient der Fixierung in dem Aufnahmeraum 77 des Aufnahmeelements 70 (Fig. 15). In der Knopfplatte 91 kann vorteilhafterweise eine einseitig offene Aushöhlung 97 angeordnet sein. Durch die Aushöhlung 97 lässt sich eine Materialanhäufung im Kunststoffbauteil der Knopfplatte vermeiden.

Aus Fig. 20 wird ersichtlich, dass der Durchmesser der Knopfplatte 91D größer ist als der Durchmesser des Halses 93D und dass der Übergang von Hals 93 zu Knopfplatte 91 an der oberen Seite gerade ist. Da der Durchmesser der Knopfplatte 91D größer ist als der Durchmesser des Halses 93D, ragt die Knopfplatte 91 über den Durchmesser des Halses 93D hinaus und es entsteht ein Überhang 92. Die Knopfplatte 91 umfasst somit den Überhang 92, der über den Umfang des Halses 93D hinausragt.

Der Durchmesser des Halses 93D wird entlang einer maximalen Länge 92L des Überhanges allmählich in den Durchmesser der Knopfplatte 91D übergeleitet. Ein Winkel δ beschreibt, in welchem Winkel der Überhang 92 zum Hals 93 angeordnet ist. Der Winkel δ kann beispielsweise 90 bis 135° betragen.

Der Hals 93 hat eine obere Länge 93L1 und eine untere Länge 93L2, wobei die obere Länge 93L1 geringer ist als die untere Länge 93L2. In der Ansicht gemäß Fig. 20 ist der Hals daher mit einem Winkel γ abgewinkelt zur Seitenwand 85 angeordnet. Der Winkel γ kann beispielsweise 90° bis 135° betragen.

Das Verbindungselement 80 hat an der dem Befestigungselement 90 gegenüberliegenden Seite eine Aussparung 82 und einen Haltesteg 83 (siehe Fig. 17 und 18). Somit liegen Aussparung 82 und Haltesteg 83 am unteren Ende 95 des Verbindungselements 80. Der Haltesteg 83 entsteht dadurch, dass die Grundfläche des Verbindungselements 80 die Aussparung 82 aufweist. Der Haltesteg ist als Teil der Seitenwand 85 des Verbindungselements 80 ausgebildet und ist am unteren Ende 95 des Verbindungselements 80 angeordnet.

Die Aussparung 82 ist derart ausgebildet, dass sie eine nicht abgebildete Bänderung 96 aufnehmen kann. Die Bänderung 96 kann mit ihrer flachen Seite durch die Aussparung und über den Haltesteg 83 verlaufen. Die Aussparung 82 ist in Größe und Form derart ausgebildet, dass die Bänderung 96 einfach und sicher durch die Aussparung hindurchgeführt werden kann. Die Aussparung 82 ist in dieser Ausführungsform als längliche Öffnung ausgebildet. Die Aussparung weist eine halbmondförmige Wölbung auf.

Die Aussparung 82 ist zwischen 80 mm und 10 mm, bevorzugt zwischen 40 mm und 15 mm beispielsweise 22 mm breit. Die Aussparung 82 ist zwischen 25 mm und 2 mm, bevorzugt zwischen 10 mm und 3 mm beispielsweise 4,1 mm hoch. Vorteilhafterweise ist die Aussparung 82 so breit ausgebildet, dass sie eine geeignete Bänderung 96 aufnehmen kann. Weiterhin ist die Aussparung 82 so hoch ausgebildet, dass die Bänderung 96 komfortabel durch die Aussparung hindurchzuführen ist.

Die Aussparung 82 ist begrenzt durch die Grundfläche des Verbindungselements 80. Am unteren Ende 95 des Verbindungselements 80 ist die Aussparung 82 begrenzt durch die Seitenwand 85. Die Seitenwand 85 am unteren Ende 95 umfasst den Haltesteg 83. Anders gesagt ist der Haltesteg 83 ausgebildet, da die Grundfläche des Verbindungselements 80 eine Aussparung 82 aufweist.

Der Haltesteg 83 ist in Größe und Form derart ausgebildet, dass die Bänderung 96 sicher gehalten werden kann. In dieser Ausführungsform ist der Haltesteg 83 aus demselben Material hergestellt wie das Verbindungselement 80. Mit anderen Worten ist der Haltesteg 83 ein Bestandteil des Verbindungselements 80.

Der Haltesteg 83 weist eine variierende Dicke auf. Der Dicke des Haltestegs liegt zwischen 10 mm und 1 mm, beispielsweise zwischen 5 mm bis 2 mm. Die Dicke des Haltestegs ist mit beispielsweise 4 mm in der Mitte am größten und nimmt jeweils zu den Seiten zu einer Breite von jeweils 2,3 mm ab. Vorteilhafterweise ist der Haltesteg 83 so breit ausgebildet, dass er der Zugkraft, die eine geeignete Bänderung 96 auf das Verbindungselement 80 ausüben kann, standhält.

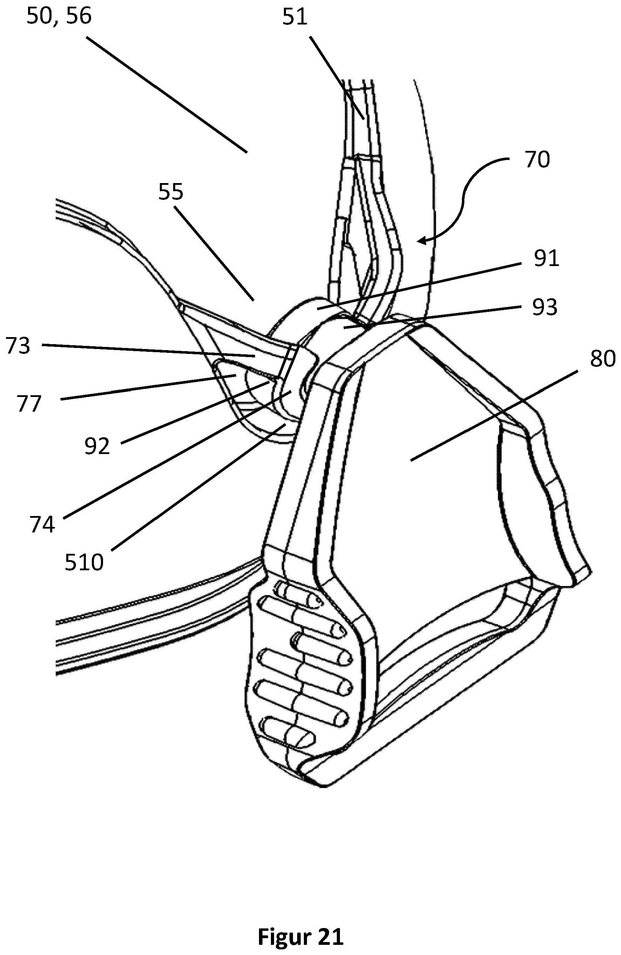

**Fig. 21** zeigt ein Verbindungselement 80, das über das Befestigungselement 90 mit dem Aufnahmeelement 70 verbunden ist. Das Aufnahmeelement 70 kann mit dem Verbindungselement 80 manuell verbunden werden. Die Verbindung von Aufnahmeelement 70 und Verbindungselement 80 ist reversibel. Das Verbindungselement 80 ist nach einer Verbindung von Aufnahmeelement 70 und Verbindungselement 80 beweglich gelagert. Das Verbindungselement 80 ist insbesondere drehbeweglich gelagert.

Durch die Führungsrinne 50 wird dem Anwender und/oder dem Patienten die Führung des Verbindungselements 80 zum Aufnahmeelement 70 ermöglicht. Das Befestigungselement 90 des Verbindungselements 80 kann beispielsweise an einem Rinneneinlauf 52 (Fig. 8) in die Führungsrinne 50 eingeführt werden. Die Knopfplatte 91 des Befestigungselements 90 kann sodann auf dem Rinnenboden 56 und an den Rinnenrändern 51 und/oder 510 (Fig. 8) entlanggeführt werden. Die Rinnenränder 51, 510 dienen dabei zur Orientierung und führen in Richtung des Endbereichs 55 der Führungsrinne, der von dem Aufnahmeelement 70 überspannt wird.

Bei Erreichen des Endbereichs 55 kann die Knopfplatte 91 des Verbindungselements 80 in den Aufnahmeraum 77 gelangen derart, dass der Hals 93 durch die Aufnahme-Öffnung X (Fig. 13/14) in den Aufnahme-Durchmesser Y (Fig. 13/14) gelangt und dort von dem Mittelsteg 74 weitestgehend umschlossen ist. Dadurch befinden sich die Knopfplatte 91 und der Überhang 92 in dem Aufnahmeraum 77, was zu einer Fixierung am Aufnahmeelement 70 führt.

Dadurch, dass die Aufnahme-Öffnung X minimal kleiner ist, als der Durchmesser des Halses 93D, und dadurch, dass das Material des Aufnahmeelements 80 etwas nachgiebig ist, kann das Befestigungselement 90 nur mit leichtem Druck durch die Aufnahme-Öffnung X hindurch in den Aufnahmeraum 77 und den Aufnahme-Durchmesser Y geführt werden. Das Einrasten des Befestigungselements 90 in das Aufnahmeelement ist spürbar und hörbar wahrzunehmen.

Sobald sich das Befestigungselement 90 in dem Aufnahmeraum 77 und dem Aufnahme-Durchmesser Y befindet, ist das Befestigungselement 90 nicht mehr ohne Kraftaufwand aus diesen zu entfernen.

Da der Aufnahme-Durchmesser Y minimal größer ist als der Durchmesser des Halses 93D des Befestigungselements 90, ist die Verbindung beweglich, insbesondere drehbeweglich gelagert.

Durch die Verbindung der mindestens 2 Verbindungselemente 90 mit dem Gesichtsteil 30 kann der Anwender und/oder der Patient die Atemmaske 100 über eine (nicht gezeigte) Bänderung 96, die sich am Verbindungselement 90 befindet, einfach, sicher und reversibel am Kopf befestigen.

Die Führungsrinne 50 bietet dem Patienten und/oder dem Anwender eine einfache und sichere Handhabung. Die Befestigung der Atemmaske 100 am Kopf gelingt auch bei eingeschränkter oder fehlender Sicht, da eine ausreichende taktile Rückmeldung über die oben ausgeführten Eigenschaften der Führungsrinne 50 gegeben ist. Über den Tastsinn sind die Orientierung der Atemmaske 100 und die Anordnung des Aufnahmeelements 80 leicht zu Erfassen.

Zudem ist die Verbindung des Verbindungselements 80 an dem Aufnahmeelement 90 einfach handhabbar und bedarf keiner besonderen Fingerfertigkeit. Der Anwender kann das Verbindungselement 80 einfach und sicher an den Griffmulden 86 greifen, entlang der Führungsrinne zum Aufnahmeelement 80 führen und dort das Befestigungselement 90 mit leichtem Druck durch die Aufnahme-Öffnung X hindurchdrücken. Hierfür muss der Anwender keine weiteren Elemente bedienen.

### Bezugszeichenliste

| | |
|---|---|
| Atemmaske | 100 |
| Schlauchanschlusssystem | 150 |
| Horizontale Ebene | 200 |
| links | 210 |
| rechts | 220 |
| Symmetrieebene | 300 |
| oben | 310 |
| unten | 320 |
| Hintere Ebene | 400 |
| hinten | 420 |
| Mittlere Ebene | 450 |
| Vordere Ebene | 500 |
| vorne | 520 |
| Stirnteil | 10 |
| Stirnpolster | 12 |
| Oberer Ankerpunkt | 13 |
| Verbindungsstelle | 14 |
| Übergangsteil | 20 |
| Gesichtsteil | 30 |
| Außenseite | 31 |
| Dichtung | 32 |
| Äußerer Rand | 33 |
| Koppelstelle | 34 |
| Zwischenglied | 35 |
| Auslass | 36 |
| Auslass-Rand | 37 |
| Einrastelement | 38 |
| Kerbelement | 39 |
| Spitze | 40 |
| Innenseite | 41 |
| Linker unterer Eckpunkt | 43 |
| Rechter unterer Eckpunkt | 44 |
| Basis | 45 |
| Führungsrinne | 50 |
| Hinterer Rinnenrand | 51 |
| Vorderer Rinnenrand | 510 |
| Rinneneinlauf | 52 |
| Rinnenkanal | 53 |
| Rinnenbucht | 54 |
| Endbereich | 55 |
| Rinnenboden | 56 |
| Überhang | 57 |
| Rinnenbreite | 58 |
| Rinnenbreite A | 58A |
| Rinnenbreite B | 58B |
| Rinnenbreite C | 58C |
| Rinnenbreite D | 58D |
| Rinnenbreite E | 58E |
| Rinnenbucht-Weite 1 | W1 |
| Rinnenbucht-Weite 2 | W2 |
| Rinnenbucht-Weite 3 | W3 |
| Winkel α | α1 |
| Winkel α | α2 |
| Winkel β | β1 |
| Winkel β | β2 |
| Vertiefung | 59 |
| Linker Ankerpunkt | 60 |
| Rechter Ankerpunkt | 61 |
| Aufnahmeelement | 70 |
| Aufnahme-Öffnung | X |
| Aufnahme-Durchmesser | Y |
| Steg | 73 |
| Steg-Ansatzfläche | 73F |
| Vorderer Steg-Ansatz | 73A |
| Hinterer Steg-Ansatz | 73B |
| Winkel ε | ε |
| Brücke | 74 |
| Mittelsteg | 75 |
| Mittelsteg-Ansatzfläche | 75F |
| Oberer Mittelsteg-Ansatz | 75A |
| Unterer Mittelsteg-Ansatz | 75B |
| Durchbruch | 76 |
| Aufnahmeraum | 77 |
| Verbindungselement | 80 |
| Äußere Grundfläche | 81 |
| Aussparung | 82 |
| Haltesteg | 83 |
| Innere Grundfläche | 84 |
| Seitenwand | 85 |
| Seitenwand-Breite B1 | B1 |
| Seitenwand-Breite B5 | B5 |
| Griffmulde | 86 |
| Griffmulde-Außenwand | 87 |
| Strukturelement | 88 |
| Griffmulde-Innenwand | 89 |
| Befestigungselement | 90 |
| Knopfplatte | 91 |
| Durchmesser der Knopfplatte | 91D |
| Länge der Knopfplatte | 91L |
| Überhang | 92 |
| Maximale Länge des Überhangs | 92L |
| Hals | 93 |
| Durchmesser des Halses | 93D |
| Oberes Ende | 94 |
| Unteres Ende | 95 |
| Bänderung | 96 |
| Aushöhlung | 97 |
| Y-Achse | Y-A |

## Patentansprüche

1. Atemmaske (100) mit einem Gesichtsteil (30), das mindestens eine Symmetrieebene (300), eine umlaufende Dichtung (32) zum Anlegen an das Gesicht eines Patienten und einen Auslass (36) umfasst, wobei das Gesichtsteil (30) eine Außenseite (31) aufweist, die bei Verwendung der Atemmaske (100) von dem Gesicht des Patienten abgewandt ist, wobei das Gesichtsteil (30) mindestens zwei Ankerpunkten (60/61) aufweist, die beidseitig der Symmetrieebene (300) angeordnet sind und wobei die mindestens zwei Ankerpunkte (60/61) jeweils ein Aufnahmeelement (70) zur lösbaren Verbindung mit einem Verbindungselement (80) umfassen und wobei das Verbindungselement (80) zur Aufnahme einer Bänderung (96) ausgebildet ist **dadurch gekennzeichnet, dass** benachbart zu dem Aufnahmeelement (70) mindestens ein Führungsbereich (50) zur Führung des Verbindungselementes (80) zu dem Aufnahmeelement (70) angeordnet ist, der durch eine Vertiefung (59) in der Außenseite (31) des Gesichtsteils (30) als Führungsrinne ausgebildet ist, wobei der Führungsbereich (50) durch Erhebungen auf der Außenseite (31) des Gesichtsteils (30) ausgebildet ist.

2. Atemmaske (100) nach einem der vorangegangenen Ansprüche **dadurch gekennzeichnet, dass** der Führungsbereich (50) beidseitig der Symmetrieebene (300) auf dem Gesichtsteil (30) angeordnet ist.

3. Atemmaske (100) nach einem der vorangegangenen Ansprüche **dadurch gekennzeichnet, dass** die Führungsrinne (50) über 5 % bis 100 % der Länge des Gesichtsteils (30) verläuft, bevorzugt über 50 % bis 100 %, beispielsweise über 95 % der Länge des Gesichtsteils (30).

4. Atemmaske (100) nach einem der vorangegangenen Ansprüche **dadurch gekennzeichnet, dass** die Führungsrinne (50) einen Rinneneinlauf (52) umfasst, der durch eine sukzessive Vertiefung einen Übergang von der Außenseite (31) zur endgültigen Vertiefung (59) bildet, wobei der Rinneneinlauf (52) an einem oberen Ende (310) des Gesichtsteils (30) angeordnet ist.

5. Atemmaske (100) nach einem der vorangegangenen Ansprüche **dadurch gekennzeichnet, dass** die Führungsrinne (50) einen Rinnenboden (56), einen hinteren Rinnenrand (51), einen vorderen Rinnenrand (510) und einen Endbereich (55) umfasst, wobei der Endbereich (55) ein Bereich des Rinnenbodens (56) ist, der von mindestens einem der Rinnenränder (51, 510) umschlossen ist.

6. Atemmaske (100) nach Anspruch 5 **dadurch gekennzeichnet, dass** die Oberfläche des Rinnenbodens (56) glatt und/oder strukturiert ausgebildet ist.

7. Atemmaske (100) nach Anspruch 5 **dadurch gekennzeichnet, dass** die Oberfläche der Rinnenränder (51, 510) glatt und/oder strukturiert ausgebildet ist.

8. Atemmaske (100) nach einem der vorangegangenen Ansprüche **dadurch gekennzeichnet, dass** die Höhe der Rinnenränder (51, 510) konstant und/oder unterschiedlich hoch ausgebildet ist, so dass die Vertiefung (59) der Führungsrinne (50) konstant und/oder unterschiedlich tief ausgebildet ist, wobei die Vertiefung (59) in einem Bereich von 0,5 mm bis 6 mm tief ausgebildet ist.

9. Atemmaske (100) nach einem der vorangegangenen Ansprüche **dadurch gekennzeichnet, dass** die Vertiefung (59) unterschiedlich tief ausgebildet ist, wobei die Vertiefung (59) im Endbereich (55) zwischen 1 mm und 6 mm tief ausgebildet ist, bevorzugt 4 mm tief, wobei die Vertiefung (59) in den anderen Bereichen der Führungsrinne (50) im Wesentlichen 0,8 mm bis 2 mm tief ausgebildet ist, bevorzugt 1 mm tief.

10. Atemmaske (100) nach einem der vorangegangenen Ansprüche **dadurch gekennzeichnet, dass** die Führungsrinne (50) unterschiedliche Rinnenbreiten (58) aufweist, wobei ein erster Abschnitt als ein Rinnenkanal (53) mit einer konstanten Rinnenbreite (58A) ausgebildet ist und wobei die Rinnenbreite (58) nach dem Rinnenkanal (53) über eine Rinnenbreite (58B) bis zu einer Rinnenbreite (58C) sukzessive zunimmt und wobei die Rinnenbreite (58) nach Erreichen der Rinnenbreite (58C) über eine Rinnenbreite (58D) bis zu einer Rinnenbreite (58E) sukzessive abnimmt.

11. Atemmaske (100) nach Anspruch 10 **dadurch gekennzeichnet, dass** im Bereich der Rinnenbreite (58C) der hintere Rinnenrand (51) maximal entfernt von der Symmetrieebene (300) angeordnet ist und der vordere Rinnenrand (510) maximal dicht an der Symmetrieebene (300) angeordnet ist.

12. Atemmaske (100) nach einem der Ansprüche 5 bis 11, **dadurch gekennzeichnet, dass** der hintere Rinnenrand (51) in einem Bereich zwischen den Rinnenbreiten (58B) und (58D) derart bogenartig zurückweicht, dass die Führungsrinne (50) in diesem Bereich als Rinnenbucht (54) ausgebildet ist, die den Endbereich (55) umfasst, wobei der Endbereich (55) maximal entfernt von der Symmetrieebene (300) angeordnet ist.

13. Atemmaske (100) nach Anspruch 12 **dadurch gekennzeichnet, dass** die Rinnenbucht (54) vom hinteren Rinnenrand (51) umschlossen ist, wobei eine Rinnenbucht-Weite (W) im horizontalen Verlauf mit zunehmender Entfernung zur Symmetrieebene sukzessive abnimmt.

14. Atemmaske (100) nach einem der Ansprüche 5 bis 13 **dadurch gekennzeichnet, dass** die Höhe des hinteren Rinnenrandes (51) im Bereich des Endbereichs (55) zunehmen kann.

15. Atemmaske (100) nach einem der Ansprüche 5 bis 14 **dadurch gekennzeichnet, dass** der Endbereich (55) der Führungsrinne (50) räumlich benachbart zu dem Aufnahmeelement (70) angeordnet ist, wobei das Aufnahmeelement (70) den Endbereich (55) der Führungsrinne (50) derart überspannt, dass ein Aufnahmeraum (77) entsteht.

16. Atemmaske (100) nach einem der vorangegangenen Ansprüche **dadurch gekennzeichnet, dass** das Aufnahmeelement (70) zwei Stege (73), eine Brücke (74), einen Aufnahme-Durchmesser (Y) und eine Aufnahme-Öffnung (X) umfasst, wobei die Brücke (74) einstückig mit den Stegen (73) gefertigt ist und wobei die Brücke (74) räumlich getrennt vom Rinnenboden (56) angeordnet ist, wobei die Stege (73), die Brücke (74) und der Rinnenboden (56) den Aufnahmeraum (77) begrenzen.

17. Atemmaske (100) nach Anspruch 16 **dadurch gekennzeichnet, dass** die Stege (73) mit einem Winkel ε zum Rinnenboden (56) angeordnet sind, wobei der Wert des Winkels ε in einem Bereich von 30° bis 90° liegt, bevorzugt in einem Bereich von 30° bis 60°.

18. Atemmaske (100) nach Anspruch 16 **dadurch gekennzeichnet, dass** die Brücke (74) zumindest abschnittsweise kreisrund ausgebildet ist mit einem Aufnahme-Durchmesser (Y) und wobei die Brücke (74) unterbrochen ist durch eine Aufnahme-Öffnung (X).

19. Atemmaske (100) nach einem der vorangegangenen Ansprüche **dadurch gekennzeichnet, dass** das Aufnahmeelement (70) mindestens einen Mittelsteg (75) und mindestens einen, bevorzugt zwei Durchbrüche (76) umfasst, wobei der Mittelsteg zwischen der Brücke (74) und der Außenseite (31) angeordnet ist.

20. Atemmaske (100) nach einem der Ansprüche 16 bis 19 **dadurch gekennzeichnet, dass** die Aufnahme-Öffnung (X) nach vorne (520) ausgerichtet ist.

21. Atemmaske (100) nach einem der vorangegangenen Ansprüche, **dadurch gekennzeichnet, dass** das Verbindungselement (80) ein lösbares Teil der Atemmaske (100) ist, wobei das Aufnahmeelement (70) eingerichtet und ausgebildet ist, das Verbindungselement (80) über ein an dem Verbindungselement (80) angeformtes Befestigungselement (90) lösbar aufzunehmen.

22. Atemmaske (100) nach einem der vorangegangenen Ansprüche, **dadurch gekennzeichnet, dass** das Verbindungselement (80) aus demselben Werkstoff wie das Gesichtsteil (30) gefertigt ist.

23. Atemmaske (100) nach einem der vorangegangenen Ansprüche, **dadurch gekennzeichnet, dass** das Verbindungselement (80) durch Aufnahme des Befestigungselements (90) in den Aufnahme-Durchmesser (Y) und den Aufnahmeraum (77) reversibel mit dem Aufnahmeelement (70) verbunden ist.

24. Atemmaske (100) nach einem der vorangegangenen Ansprüche, **dadurch gekennzeichnet, dass** die Verbindung des Verbindungselements (80) an das Aufnahmeelement (70) beweglich gelagert ist, insbesondere drehbeweglich.

## Claims

1. A breathing mask (100) with a face part (30), which comprises at least one plane of symmetry (300), a circumferential seal (32) for placing on the face of a patient, and an outlet (36), wherein the face part (30) has an outer side (31) that faces away from the face of the patient during use of the breathing mask (100), wherein the face part (30) has at least two anchor points (60/61) that are arranged on both sides of the plane of symmetry (300), and wherein the at least two anchor points (60/61) each comprise a receiving element (70) for detachably connecting to a connecting element (80) and wherein the connecting element (80) is designed to receive a strap (96), **characterized in that** at least one guide area (50) for guiding the connecting element (80) to the receiving element (70) is arranged adjacent to the receiving element (70), which guide area is formed by an indentation (59) in the outer side (31) of the face part (30) as a guide groove, wherein the guide area (50) is formed by elevations on the outer side (31) of the face part (30).

2. The breathing mask (100) according to one of the preceding claims, **characterized in that** the guide area (50) is arranged on the face part (30) on both sides of the plane of symmetry (300).

3. The breathing mask (100) according to one of the preceding claims, **characterized in that** the guide groove (50) runs over 5% to 100% of the length of the face part (30), preferably over 50% to 100%, for example over 95% of the length of the face part (30).

4. The breathing mask (100) according to one of the preceding claims, **characterized in that** the guide groove (50) comprises a groove run-in region (52), which, due to a successive indentation, forms a transition from the outer side (31) to the final indentation (59), wherein the groove run-in region (52) is arranged at an upper end (310) of the face part (30).

5. The breathing mask (100) according to one of the preceding claims, **characterized in that** the guide groove (50) comprises a groove floor (56), a rear groove edge (51), a front groove edge (510), and an end area (55), wherein the end area (55) is an area of the groove floor (56) that is surrounded by at least one of the groove edges (51, 510).

6. The breathing mask (100) according to claim 5, **characterized in that** the surface of the groove floor (56) is smooth and/or structured.

7. The breathing mask (100) according to claim 5, **characterized in that** the surface of the groove edges (51, 510) is smooth and/or structured.

8. The breathing mask (100) according to one of the preceding claims, **characterized in that** the height of the groove edges (51, 510) is constant and/or of different heights, such that the indentation (59) of the guide groove (50) is constant and/or of different depths, wherein the indentation (59) is in a range from 0.5 mm to 6 mm deep.

9. The breathing mask (100) according to one of the preceding claims, **characterized in that** the indentation (59) is of different depths, wherein the indentation (59) in the end area (55) is between 1 mm and 6 mm deep, preferably 4 mm deep, wherein the indentation (59) in the other areas of the guide groove (50) is substantially 0.8 mm to 2 mm deep, preferably 1 mm deep.

10. The breathing mask (100) according to one of the preceding claims, **characterized in that** the guide groove (50) has different groove widths (58), wherein a first portion is designed as a groove channel (53) with a constant groove width (58A) and wherein the groove width (58) after the groove channel (53) increases successively through a groove width (58B) to a groove width (58C) and wherein, after reaching the groove width (58C), the groove width (58) decreases successively through a groove width (58D) to a groove width (58E).

11. The breathing mask (100) according to claim 10, **characterized in that,** in the area of the groove width (58C), the rear groove edge (51) is arranged at a maximum distance from the plane of symmetry (300) and the front groove edge (510) is arranged maximally close to the plane of symmetry (300).

12. The breathing mask (100) according to one of claims 5 to 11, **characterized in that,** in an area between the groove widths (58B) and (58D), the rear groove edge (51) recedes in an arc shape such that the guide groove (50) is designed in this area as a groove protrusion (54) which comprises the end area (55), wherein the end area (55) is arranged at a maximum distance from the plane of symmetry (300).

13. The breathing mask (100) according to claim 12, **characterized in that** the groove protrusion (54) is surrounded by the rear groove edge (51), wherein a horizontal course of a groove protrusion width (W) decreases successively as the distance from the plane of symmetry increases.

14. The breathing mask (100) according to one of claims 5 to 13, **characterized in that** the height of the rear groove edge (51) can increase in the area of the end area (55).

15. The breathing mask (100) according to one of claims 5 to 14, **characterized in that** the end area (55) of the guide groove (50) is arranged spatially adjacent to the receiving element (70), wherein the receiving element (70) spans the end area (55) of the guide groove (50) such that a receiving space (77) is formed.

16. The breathing mask (100) according to one of the preceding claims, **characterized in that** the receiving element (70) comprises two webs (73), a bridge (74), a receiving diameter (Y), and a receiving opening (X), wherein the bridge (74) is manufactured in one piece with the webs (73) and wherein the bridge (74) is arranged spatially separate from the groove floor (56), wherein the webs (73), the bridge (74), and the groove floor (56) delimit the receiving space (77).

17. The breathing mask (100) according to claim 16, **characterized in that** the webs (73) are arranged at an angle ε to the groove floor (56), wherein the value of the angle ε is in a range from 30° to 90°, preferably in a range from 30° to 60°.

18. The breathing mask (100) according to claim 16, **characterized in that** the bridge (74) is circular at least in portions with a receiving diameter (Y) and wherein the bridge (74) is interrupted by a receiving opening (X).

19. The breathing mask (100) according to one of the preceding claims, **characterized in that** the receiving element (70) comprises at least one central web (75) and at least one, preferably two cut-outs (76), wherein the central web is arranged between the bridge (74) and the outer side (31).

20. The breathing mask (100) according to one of claims 16 to 19, **characterized in that** the receiving opening (X) is oriented forward (520).

21. The breathing mask (100) according to one of the preceding claims, **characterized in that** the connecting element (80) is a detachable part of the breathing mask (100), wherein the receiving element (70) is configured and designed to detachably receive the connecting element (80) via a fastening element (90) formed on the connecting element (80).

22. The breathing mask (100) according to one of the preceding claims, **characterized in that** the connecting element (80) is manufactured from the same material as the face part (30).

23. The breathing mask (100) according to one of the preceding claims, **characterized in that** the connecting element (80) is reversibly connected to the receiving element (70) by receiving the fastening element (90) in the receiving diameter (Y) and the receiving space (77).

24. The breathing mask (100) according to one of the preceding claims, **characterized in that** the connection of the connecting element (80) to the receiving element (70) is mounted in a movable manner, in particular rotationally movable.

## Revendications

1. Masque respiratoire (100) doté d'une pièce faciale (30) comportant au moins un plan de symétrie (300), un joint périphérique (32) destiné à être appliqué sur le visage d'un patient et une sortie (36), dans lequel la pièce faciale (30) présente un côté extérieur (31), lequel est opposé au visage du patient pendant l'utilisation du masque respiratoire (100), dans lequel la pièce faciale (30) présente au moins deux points d'ancrage (60/61) disposés de part et d'autre du plan de symétrie (300) et dans lequel les au moins deux points d'ancrage (60/61) comportent respectivement un élément de réception (70) pour le raccordement détachable à un élément de liaison (80) et dans lequel l'élément de liaison (80) est conçu pour recevoir un harnais (96), **caractérisé en ce qu'**au moins une zone de guidage (50) réalisée comme une gouttière de guidage par un évidement (59) dans le côté extérieur (31) de la pièce faciale (30) est disposée de façon adjacente à l'élément de réception (70) pour le guidage de **l'élément** de liaison (80) vers **l'élément** de réception (70), dans lequel la zone de guidage (50) est réalisée par des saillies sur le côté extérieur (31) de la pièce faciale (30).

2. Masque respiratoire (100) selon l'une des revendications précédentes, **caractérisé en ce que** la zone de guidage (50) est disposée de part et d'autre du plan de symétrie (300) sur la pièce faciale (30).

3. Masque respiratoire (100) selon l'une des revendications précédentes, **caractérisé en ce que** la gouttière de guidage (50) s'étend sur 5 % à 100 % de la longueur de la pièce faciale (30), de préférence sur 50 % à 100 %, par exemple sur 95 % de la longueur de la pièce faciale (30).

4. Masque respiratoire (100) selon l'une des revendications précédentes, **caractérisé en ce que** la gouttière de guidage (50) comporte une entrée de gouttière (52), laquelle forme une transition entre le côté extérieur (31) et l'évidement final (59) par un évidement progressif, dans lequel l'entrée de gouttière (52) est disposée à une extrémité supérieure (310) de la pièce faciale (30).

5. Masque respiratoire (100) selon l'une des revendications précédentes, **caractérisé en ce que** la gouttière de guidage (50) comporte un fond de gouttière (56), un bord de gouttière arrière (51), un bord de gouttière avant (510) et une région d'extrémité (55), dans lequel la région d'extrémité (55) est une région du fond de gouttière (56) entourée par l'un au moins des bords de gouttière (51, 510).

6. Masque respiratoire (100) selon la revendication 5, **caractérisé en ce que** la surface du fond de gouttière (56) est réalisée de façon lisse et/ou structurée.

7. Masque respiratoire (100) selon la revendication 5, **caractérisé en ce que** la surface des bords de gouttière (51, 510) est réalisée de façon lisse et/ou structurée.

8. Masque respiratoire (100) selon l'une des revendications précédentes, **caractérisé en ce que** la hauteur des bords de gouttière (51, 510) est réalisée de façon constante et/ou variable, de sorte que l'évidement (59) de la gouttière de guidage (50) est réalisé avec une profondeur constante et/ou variable, dans lequel l'évidement (59) est réalisé dans une plage de profondeur de 0,5 mm à 6 mm.

9. Masque respiratoire (100) selon l'une des revendications précédentes, **caractérisé en ce que** l'évidement (59) est réalisé avec une profondeur variable, dans lequel l'évidement (59) est réalisé avec une profondeur comprise entre 1 mm et 6 mm dans la région d'extrémité (55), de préférence une profondeur de 4 mm, dans lequel l'évidement (59) est réalisé essentiellement avec une profondeur de 0,8 mm à 2 mm, de préférence de 1 mm, dans les autres régions de la gouttière de guidage (50).

10. Masque respiratoire (100) selon l'une des revendications précédentes, **caractérisé en ce que** la gouttière de guidage (50) présente des largeurs de gouttière (58) variables, dans lequel une première section est réalisée comme un canal de gouttière (53) avec une largeur de gouttière constante (58A) et dans lequel la largeur de gouttière (58) augmente progressivement après le canal de gouttière (53) jusqu'à une largeur de gouttière (58C) en passant par une largeur de gouttière (58B) et dans lequel la largeur de gouttière (58) diminue progressivement après avoir atteint la largeur de gouttière (58C), jusqu'à une largeur de gouttière (58E) en passant par une largeur de gouttière (58D).

11. Masque respiratoire (100) selon la revendication 10, **caractérisé en ce que** dans la région de la largeur de gouttière (58C), le bord de gouttière arrière (51) est disposé à une distance maximale du plan de symétrie (300) et le bord de gouttière avant (510) est disposé au plus près du plan de symétrie (300).

12. Masque respiratoire (100) selon l'une des revendications 5 à 11, **caractérisé en ce que** le bord de gouttière arrière (51) recule de façon arquée dans une région entre les largeurs de gouttière (58B) et (58D), de sorte que dans cette région, la gouttière de guidage (50) est réalisée comme une baie de gouttière (54) comportant la région d'extrémité (55), dans lequel la région d'extrémité (55) est disposée à une distance maximale du plan de symétrie (300).

13. Masque respiratoire (100) selon la revendication 12, **caractérisé en ce que** la baie de gouttière (54) est entourée par le bord de gouttière arrière (51), dans lequel une largeur de baie de gouttière (W) diminue progressivement sur son tracé horizontal à mesure que la distance par rapport au plan de symétrie augmente.

14. Masque respiratoire (100) selon l'une des revendications 5 à 13, **caractérisé en ce que** la hauteur du bord de gouttière arrière (51) peut augmenter dans la région de la région d'extrémité (55).

15. Masque respiratoire (100) selon l'une des revendications 5 à 14, **caractérisé en ce que** la région d'extrémité (55) de la gouttière de guidage (50) est disposée de façon spatialement adjacente à l'élément de réception (70), dans lequel l'élément de réception (70) couvre la région d'extrémité (55) de la gouttière de guidage (50) de manière à créer un espace de réception (77).

16. Masque respiratoire (100) selon l'une des revendications précédentes, **caractérisé en ce que** l'élément de réception (70) comporte deux âmes (73), un pont (74), un diamètre de réception (Y) et une ouverture de réception (X), dans lequel le pont (74) est conçu d'un seul tenant avec les âmes (73) et dans lequel le pont (74) est disposé de façon spatialement séparée du fond de gouttière (56), dans lequel les âmes (73), le pont (74) et le fond de gouttière (56) délimitent d'espace de réception (77).

17. Masque respiratoire (100) selon la revendication 16, **caractérisé en ce que** les âmes (73) sont disposées sous un angle ε par rapport au fond de gouttière (56), dans lequel la valeur de l'angle ε est comprise dans une plage de 30° à 90°, de préférence dans une plage de 30° à 60°.

18. Masque respiratoire (100) selon la revendication 16, **caractérisé en ce que** le pont (74) est réalisé au moins par sections de façon circulaire avec un diamètre de réception (Y), et dans lequel le pont (74) est interrompu par une ouverture de réception (X).

19. Masque respiratoire (100) selon l'une des revendications précédentes, **caractérisé en ce que** l'élément de réception (70) comporte au moins une âme centrale (75) et au moins une percée (76), de préférence deux, dans lequel l'âme centrale est disposée entre le pont (74) et le côté extérieur (31).

20. Masque respiratoire (100) selon l'une des revendications 16 à 19, **caractérisé en ce que** l'ouverture de réception (X) est orientée vers l'avant (520).

21. Masque respiratoire (100) selon l'une des revendications précédentes, **caractérisé en ce que** l'élément de liaison (80) est une pièce détachable du masque respiratoire (100), dans lequel l'élément de réception (70) est configuré et réalisé pour recevoir l'élément de liaison (80) de façon détachable par le biais d'un élément de fixation (90) formé sur l'élément de liaison (80).

22. Masque respiratoire (100) selon l'une des revendications précédentes, **caractérisé en ce que** l'élément de liaison (80) est conçu à partir du même matériau que la pièce faciale (30).

23. Masque respiratoire (100) selon l'une des revendications précédentes, **caractérisé en ce que** l'élément de liaison (80) est relié de façon réversible à l'élément de réception (70) par réception de l'élément de fixation (90) dans le diamètre de réception (Y) et l'espace de réception (77).

24. Masque respiratoire (100) selon l'une des revendications précédentes, **caractérisé en ce que** le raccordement de l'élément de liaison (80) à l'élément de réception (70) est monté de façon mobile, en particulier mobile en rotation.
